(19) Europäisches Patentamt
European Patent Office
Office européen des brevets

(11) **EP 2 273 969 B1**

(12) **EUROPEAN PATENT SPECIFICATION**

(45) Date of publication and mention of the grant of the patent:
**25.12.2019 Bulletin 2019/52**

(21) Application number: **09742775.1**

(22) Date of filing: **30.04.2009**

(51) Int Cl.:
***A61K 8/68** (2006.01)* ***A61K 8/65** (2006.01)*
***A61K 8/73** (2006.01)* ***A61K 8/92** (2006.01)*
***A61K 31/164** (2006.01)* ***A61K 31/715** (2006.01)*
***A61K 31/728** (2006.01)* ***A61K 38/17** (2006.01)*
***A61P 17/16** (2006.01)* ***A61Q 19/00** (2006.01)*

(86) International application number:
**PCT/JP2009/058792**

(87) International publication number:
**WO 2009/136651 (12.11.2009 Gazette 2009/46)**

(54) **TOPICAL COMPOSITION FOR EXTERNAL USE AND PROCESS FOR PRODUCING THE SAME**

TOPISCHE ZUSAMMENSETZUNG ZUR EXTERNEN ANWENDUNG UND VERFAHREN ZU IHRER HERSTELLUNG

COMPOSITION TOPIQUE POUR UNE UTILISATION EXTERNE ET SON PROCÉDÉ DE FABRICATION

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **09.05.2008 JP 2008123688**

(43) Date of publication of application:
**19.01.2011 Bulletin 2011/03**


(73) Proprietor: **FUJIFILM Corporation**
**Tokyo 106-8620 (JP)**

(72) Inventors:
- **KUBO, Toshiaki**
**Ashigarakami-gun**
**Kanagawa 258-8577 (JP)**
- **TASHIRO, Tomoko**
**Ashigarakami-gun**
**Kanagawa 258-8577 (JP)**
- **MORI, Hisahiro**
**Ashigarakami-gun**
**Kanagawa 258-8577 (JP)**
- **ARAKAWA, Jun**
**Ashigarakami-gun**
**Kanagawa 258-8577 (JP)**

(74) Representative: **Klunker IP**
**Patentanwälte PartG mbB**
**Destouchesstraße 68**
**80796 München (DE)**


(56) References cited:
**EP-A2- 1 250 907** **WO-A1-2007/004300**
**JP-A- 2002 317 124** **JP-A- 2003 113 393**
**US-A1- 2006 057 091**

- **DATABASE WPI Week 200321 Thomson Scientific, London, GB; AN 2003-214367 XP002716705, & JP 2002 338459 A (NIPPON SURFACTANT KOGYO KK) 27 November 2002 (2002-11-27)**
- **DATABASE WPI Week 199337 Thomson Scientific, London, GB; AN 1993-293214 XP002716706, & KR 920 010 246 B (KOREA COSMETICS CO) 21 November 1992 (1992-11-21)**
- **DATABASE WPI Week 200825 Thomson Scientific, London, GB; AN 2008-D46131 XP002716707, & KR 100 693 292 B1 (BIOLAND LTD) 13 March 2007 (2007-03-13)**


Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).


EP 2 273 969 B1

## Description

### Technical Field

The present invention relates to a topical composition for external use containing a ceramide analogue-containing particle, and a process for producing a topical composition for external use.

### Background Art

Ceramide is present in a stratum corneum of a skin, and plays an important role for constructing a necessary lipid barrier for retaining a moisture, and maintaining a moisture. Ceramide present in a stratum conrneum is produced by degradation of cerebroside with an enzyme called cerebrosidase. It is known that a part of ceramide is changed into phytosphingosine and sphingosine with an enzyme called ceramidase, and they are important as an agent of regulating proliferation and differentiation of cells. In a human skin, six kinds of different types of ceramides are present, and have different functions, respectively.

However, since ceramide is a substance having high crystallizability, has low solubility in other oil solution, and precipitates a crystal at a low temperature, it was difficult to maintain stability when incorporated into cosmetics.

As a composition containing ceramides, an emulsion composition including specified sphingoglycolipids having the moisturizing activity, the skin roughness preventing activity and the emulsifying activity is disclosed (see e.g. Japanese Patent Application Laid-Open (JP-A) No. 2000-51676).

In addition, a ceramide-incorporated cosmetic additive containing cholesterol, fatty acid, and a water-soluble polymer (see e.g. JP-A No. 7-187987), and a water-in-oil emulsion composition in which a salt formed of sphingosines and a specified fatty acid is used as an emulsifying agent, and an oil-soluble antioxidant is added at a specified ratio, as a topical composition for external use, which is excellent in stability also in the case of severe temperature change, and is good in a use feeling (see e.g. JP-A No. 2006-335692) are disclosed.

In addition, as the technique for formulation into preparations, a process for producing an additive for cosmetics, in which a crude dispersion of sphingoglycolipid is subjected to finely-dividing treatment using a predetermined jet stream in order to sufficiently exert the emollient effect of sphingoglycolipid is disclosed (see e.g. JP-ANo. 11-310512).

In addition, as the technique for solubilizing ceramides transparent, and stably incorporating them, incorporation of specified fatty acid and specified surfactant is disclosed (see e.g. JP-ANos. 2001-139796 and 2001-316217). However, in order to solubilize ceramides transparent, it is necessary to increase incorporation of a surfactant and, for this reason, safety and a use feeling are deteriorated, in some cases. On the other hand, when an amount of a surfactant to be incorporated is reduced in order to obtain the excellent use feeling, a preparation becomes cloudy to translucent milky in many cases, and ceramide may not be solubilized transparent and, in this case, separation and creaming are caused with time, and it is difficult to maintain sufficient stability with time.

Like this, even these techniques are not sufficient to satisfy the high demand on the emollient effect in recent years, without deteriorating stability of a ceramide analogue in a composition.

### DISCLOSURE OF THE INVENTION

### Problems to be Solved by the Invention

An object of the present invention is to provide a topical composition for external use containing a ceramide analogue-containing particle of a minute particle diameter uniformly and stably, and a process for producing a topical composition for external use in which dispersion stability of a ceramide analogue-containing particle is good.

### Means to Solve the Problem

Specific means for attaining the above object are as follows.

<1> A topical composition for external use comprising: a ceramide analogue-containing particle having a particle diameter of 1 nm to 50 nm; and a water-soluble polymer, wherein the ceramide analogue is a combination of phytosphingosine and two kinds of natural ceramides.

<2> The topical composition for external use of <1>, further comprising an oil component different from the ceramide analogue in an amount of 20 parts by mass or less relative to 1 part by mass of the ceramide analogue-containing particle.

<3> The topical composition for external use of <2>, wherein the content of the oil component different from the ceramide analogue-containing particle is 10 parts by mass or less relative to 1 part by mass of the ceramide analogue-containing particle.

<4> The topical composition for external use of <1>, wherein the water-soluble polymer is a natural polymer.

<5> The topical composition for external use of <1>, wherein the water-soluble polymer is a collagen derivative, wherein the collagen derivative is at least one selected from a hydrolyzed collagen or a water-soluble collagen.

<6> The topical composition for external use of <5>, wherein the weight-average molecular weight of the collagen derivative is 5,000 or less.

<7> The topical composition for external use of <1>, wherein the water-soluble polymer is a polysaccharide.

<8> The topical composition for external use of <7>, wherein the weight-average molecular weight of the polysaccharide is 100,000 or less.

<9> The topical composition for external use of <1>, wherein the water-soluble polymer is hyaluronic acid.

<10> The topical composition for external use of <9>, wherein the weight-average molecular weight of the hyaluronic acid is 300,000 or less.

<11> The topical composition for external use of <1>, wherein the ceramide analogue-containing particle is formed in the presence of a water-soluble polymer. <12> A process for producing a topical composition for external use containing a ceramide analogue-containing particle having a particle diameter of 1 nm to 50 nm; and a water-soluble polymer, the process comprising forming a ceramide analogue-containing particle in an aqueous phase containing a water-soluble polymer, wherein the ceramide analogue is a combination of phytosphingosine and two kinds of natural ceramides.<13> The process for producing a topical composition for external use of <12>, wherein a viscosity of the aqueous phase is 30 mPa·s or less.

Effects of the Invention

According to the present invention, a topical composition for external use containing a ceramide analogue-containing particle of a minute particle diameter uniformly and stably, and a process for producing a topical composition for external use in which dispersion stability of a ceramide analogue-containing particle is good may be provided.

BRIEF DESCRIPTION OF THE DRAWINGS

Fig. 1 is an exploded perspective of a microdevice as one example of a micromixer.

Fig. 2 is a schematic cross-sectional view of a T-type microreactor showing one example of a mixing mechanism with a T-type microreactor.

Fig. 3 is a concept view of a T-type microreactor showing one example of a mixing mechanism with a T-type microreactor.

BEST MODE FOR CARRYING OUT THE INVENTION

<Topical composition for external use>

The topical composition for external use of the invention contains a ceramide analogue-containing particle of a particle diameter of 1 nm to 50 nm, and a water-soluble polymer, wherein the ceramide analogue is a combination of ceramide 3, ceramide 6 and phytosphingosine and two kinds of natural ceramides.

The invention has the great characteristic that the ceramide analogue is contained at a shape of a fine dispersed particle.

Such the ceramide analogue-containing particle may be dispersed as it is in an aqueous phase component containing a water-soluble polymer, or may form an oily phase as a dispersed particle with an oil component different from the ceramide analogue, and take a form of an emulsion.

Such the oil component is contained preferably in an amount of 20 parts by mass or less, further preferably 10 parts by mass or less relative to 1 part by mass of the ceramide analogue-containing particle.

Various components contained in the topical composition for external use of the invention will be sequentially explained below.

[Water-soluble polymer]

As the water-soluble-polymer used in the invention, any water-soluble polymer may be used as far as it is a polymer which is dissolved in water (25°C) in an amount of at least around 0.001% by mass.

Examples of the water-soluble polymer which may be used in the invention include:

polysaccharides such as pectin, kappa carrageenan, locust bean gum, guar gum, hydroxypropylguar gum, xanthan gum, karaya gum, tamarind seed polysaccharide, arabic gum, tragacanth gum, sodium hyaluronate, sodium chondroitin sulfate, sodium alginate etc.;
proteins such as casein, albumin, methylated collagen, hydrolyzed collagen, water-soluble collagen etc.;
synthetic polymers such as carboxyvinyl polymer, poly(sodium acrylate), polyvinyl alcohol, polyethylene glycol, ethylene oxide/propylene oxide block copolymer etc.;
water-soluble cellulose derivatives such as hydroxyethylcellulose, hydroxymethylcellulose etc.

The water-soluble polymer which may be used in the invention may be synthetic or natural and, among the water-soluble polymer, a natural polymer is preferable and polysaccharides and proteins which are naturally occurring polymers are preferably used.

More preferable examples include collagen derivatives, polysaccharides, and hyaluronic acids and, from a viewpoint of stabilization of the ceramide analogue particle and step suitability described later, as the collagen derivatives, the weight-average molecular weight of 5,000 or less is preferable, and the range of 200 to 3,000 is more preferable. In addition, preferable examples of the polysaccharides include specifically xanthan gum, kappa carrageenan, and dextran, and the weight-average molecular weight of 3,000,000 or less is preferable, and the more preferable molecular weight is in the range of 10,000 to 2,000,000. As the hyaluronic acids, the weight-average molecular weight of 300,000 or less is preferably used, and the more preferable molecular weight is in the rage of 5,000 to 200,000.

As the weight-average molecular weight of these polymers, a value measured by gel permeation chromatography is used.

In the topical composition for external use of the invention, as the water-soluble polymer, only one kind may be used, or two or more kinds may be used together depending on the purpose.

The content of the water-soluble polymer in the topical composition for external use is preferably in the range of 0.001 to 5% by mass, more preferably in the rage of 0.01 to 1% by mass.

Within the range of the content, the composition has an advantage that the composition is not sticky, and a good feeling is obtained.

[Aqueous component]

The composition of external use of the invention has a feature that the ceramide analogue-containing particle detailed below is dispersed in an aqueous phase containing the water-soluble polymer. As the aqueous phase, an aqueous solution containing water as a main component, which contains the water-soluble polymer, may be used. A water-soluble functional component such as a water-soluble antioxidant, and a plant extract may be further added to the aqueous phase in such the range that the effect of the invention is not deteriorated.

[Ceramide analogue]

The ceramide analogue in the invention is a combination of phytosphingosine and two kinds of natural ceramides, wherein the ceramide may be any of natural ceramide regardless of their origin such as synthetic and extract, and glycosylated ceramide such as sphingoglycolipid, and an analogue thereof. Examples of the ceramide which may be used in the invention include compounds which are generally known as ceramide 1, ceramide 2, ceramide 3, ceramide 4, ceramide 5, ceramide 6, sphingosine, phytosphingosine, or glycosylated ceramide.

The invention has a feature that the ceramide analogue is contained in the topical composition for external use in the shape of a particle containing the ceramid analogue. Such the ceramide analogue-containing particle may be dispersed as it is in an aqueous phase component containing the water-soluble polymer, or may form an oily phase as a dispersed particle with an oil component different from the ceramide analogue, and may be dispersed as an oily phase particle in the aqueous phase. A method of forming the ceramide analogue-containing particle will be described later.

The ceramide analogue which may be used in the invention will be explained in detail below.

(Natural ceramides)

Examples of a fundamental structural formula of a natural ceramide which may be preferably used in the ceramide analogue in the invention are shown in (1-1) to (1-10). (1-1) is a compound known as ceramide 1, (1-2) is a compound known as ceramide 9, (1-3) is a compound known as ceramide 4, (1-4) is a compound known as ceramide 2, (1-5) is a compound known as ceramide 3, (1-6) is a compound known as ceramide 5, (1-7) is a compound known as ceramide 6, (1-8) is a compound known as ceramide 7, (1-9) is a compound known as ceramide 8, and (1-10) is a compound known as ceramide 3B.

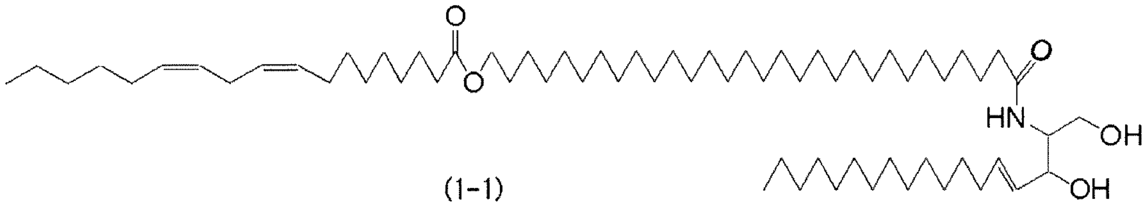

(1-1)

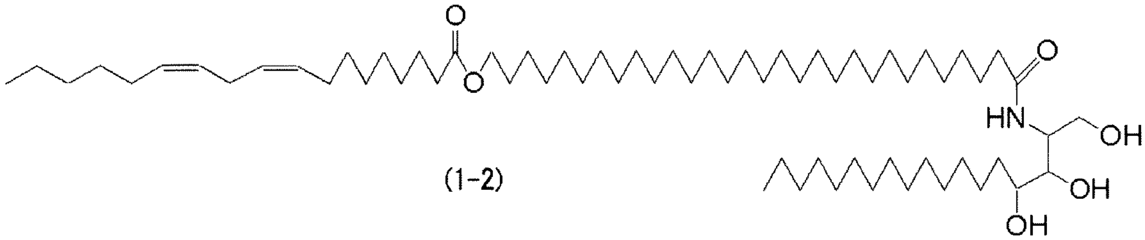

(1-2)

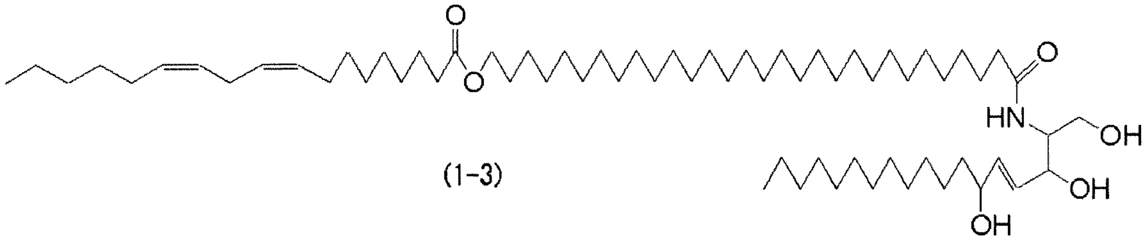

(1-3)

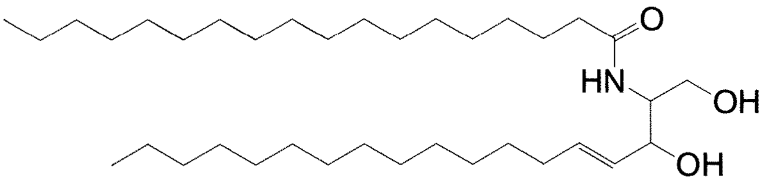

(1-4)

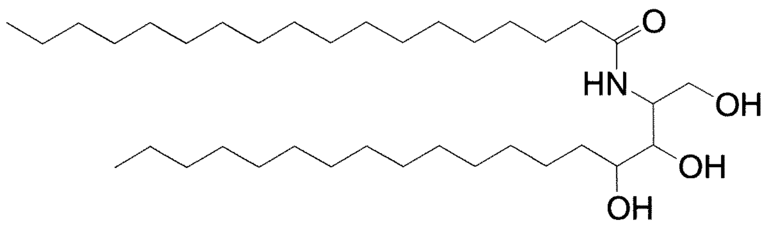

(1-5)

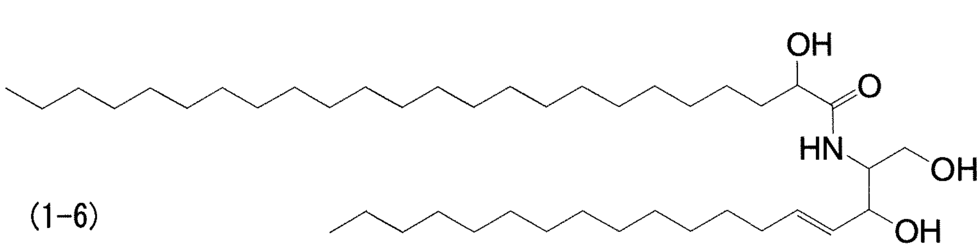

(1-6)

(1-7)

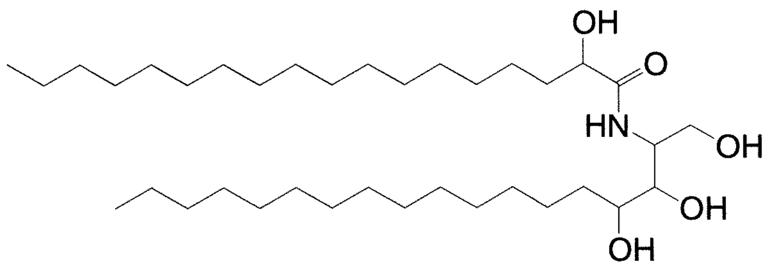

(1-8)

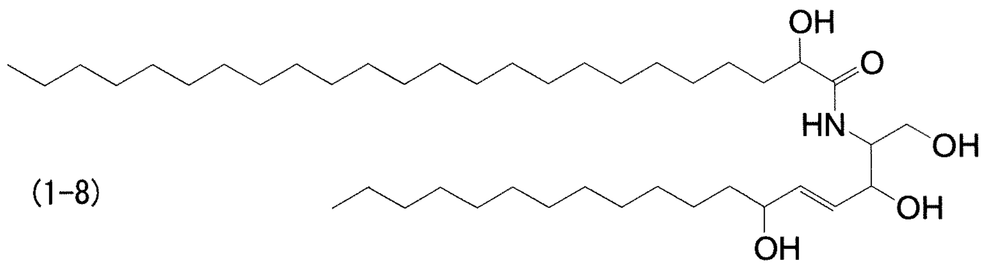

(1-9)

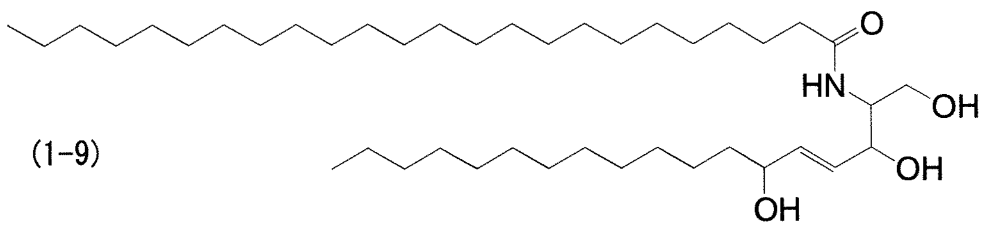

(1-10)

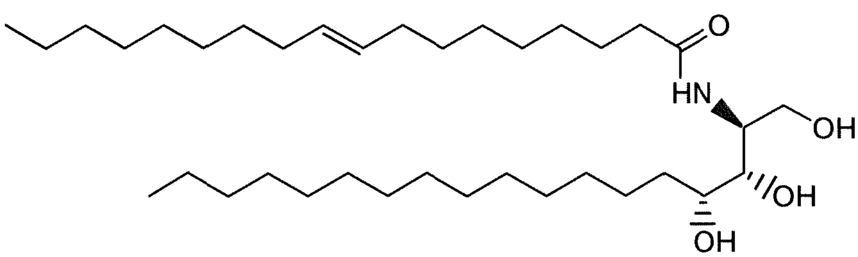

The above structural formula shows one example of each ceramide. Since ceramide is a natural substance, in ceramides actually derived from a human or an animal, there are various variation examples in the length of the alkyl chain, and ceramide having the above skeleton may have any structure in the alkyl chain length.

Alternatively, ceramides modified depending on the purpose such as ceramide in which a double bond is introduced in the molecule in order to impart solubility for the purpose of formulation into preparations, and ceramide in which a hydrophobic group is introduced to impart permeability, may be used.

These ceramides having the general structure named natural type may be a natural product (extract) or a synthetic substance, or commercially available ceramide may be used depending on the purpose.

As these ceramides, a natural (D(-) body) optically active body may be used, or a non-natural (L(+) body) optically active body may be used, or further, a mixture of a natural type and a non-natural type may be used. A relative configuration of the above compounds may be natural configuration, or other non-natural configuration, or a mixture thereof.

When the topical composition for external use is used for the purpose of an emollient of a skin, from a viewpoint of the barrier effect, it is preferable to use the natural optically active body.

Such the natural ceramides are also available as a sold product, and examples include Ceramide I, Ceramide III, Ceramide IIIA, Ceramide IIIB, Ceramide IIIC, and Ceramide VI (all manufactured by Cosmofarm), Ceramide TIC-001 (manufactured by Takasago International Corporation), CERAMIDE II (manufactured by Quest International), DS-Ceramide VI, DS-CLA-Phytoceramide, C6-Phytoceramide, and DS-ceramide Y3S (manufactured by DOOSAN), and CERAMIDE2 (manufactured by Sedama), and the exemplified compound (1-5) is available as trade name: CERAMIDE 3, manufactured by Evonik (formerly Deggusa), and the exemplified compound (1-7) is available as trade name: CERAMIDE 6, manufactured by Evonik (formerly Deggusa).

(Glycosylated ceramide)

Other preferable examples of the ceramide analogue in the invention include glycosylated ceramide compound (hereinafter, also referred to as "sugar ceramide compound") containing sugars in the molecule.

Examples of the sugar used in modification of ceramide include monosaccharides such as glucose, and galactose, disaccharides such as lactose, and maltose and, further, oligosaccharides and polysaccharides obtained by polymerizing these monosaccharides and disaccharides with a glucoside bond. Alternatively, glycosylated ceramide may be a sugar derivative in which a hydroxyl group in a unit of sugar is replaced with other group. Such the sugar derivative includes glucosamine, glucuronic acid, and N-acetylglucosamine.

Among them, from a viewpoint of dispersion stability, sugars having the number of sugar units of 1 to 5 is preferable, and specifically, glucose and lactose are preferable, and glucose is more preferable.

Examples of the sugar ceramide compound which may be used in the invention may include the following compounds.

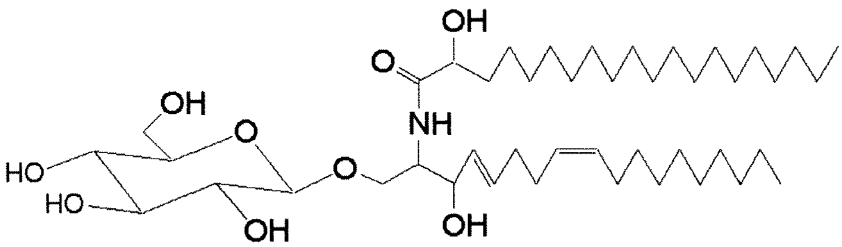

(4-1)

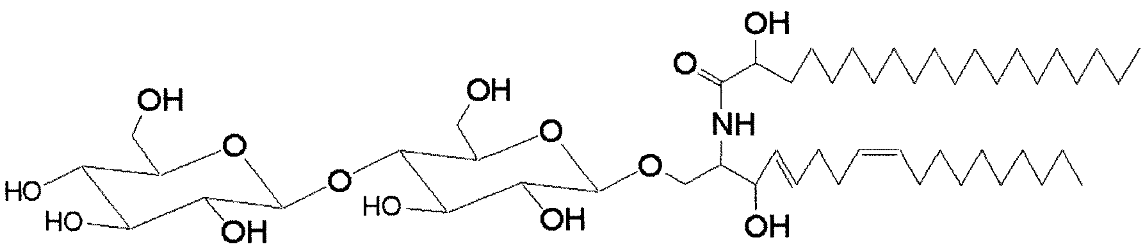

(4-2)

The sugar ceramide compound is available by synthesis or as a sold product. For example, the exemplified compound (4-1) is available as trade name: KOME SHINGOGLYCOLIPID manufactured by Okayasu Shoten Co., Ltd..

(Ceramide analogue)

As the ceramide analogue in the invention, a ceramide analogue synthesized by mimicking a structure of ceramides may be also used.

As the known compound of such the ceramide analogue, for example, a ceramide analogue represented by the following structural formula may be also used.

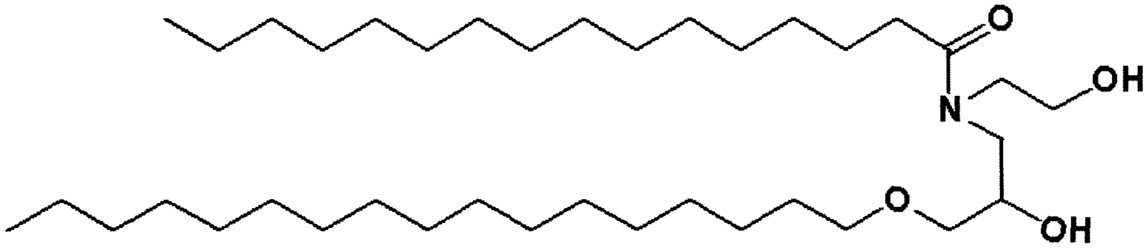

Such the ceramide analogue, for example, from a viewpoint of a use feeling and a moisturizing feeling upon use of the composition for use of the invention as cosmetics, is preferably an analogue of natural ceramide or glycosylated ceramide, more preferably an analogue of natural ceramide.

(Phytosphingosine)

As phytosphingosine in the invention, whether a synthetic product or a natural product, may be used, and such the compound is included in the ceramide analogue of the invention.
Examples include phytosphingosine, dehydrophytosphingosine, and an N-alkylated body (e.g. N-methylated body) thereof, and an acetylated body thereof.

Among them, examples of phytosphingosine which may be preferably used in the invention include PHYTOSPHINGOSINE(INCI name; 8th Edition) and exemplified compounds described below.

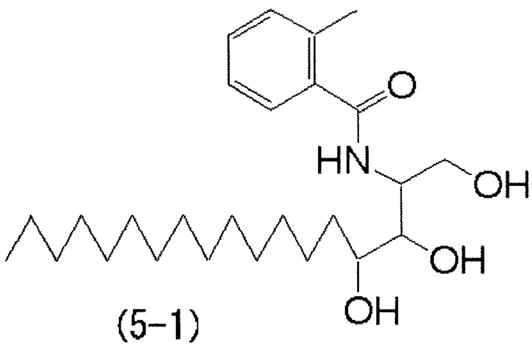

(5-1)

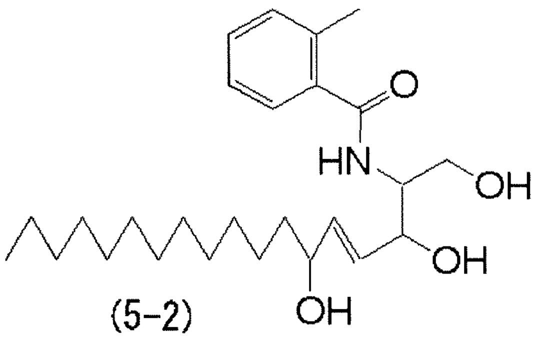

(5-2)

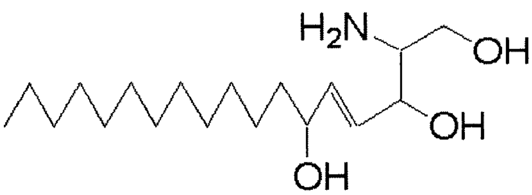

(5-3)

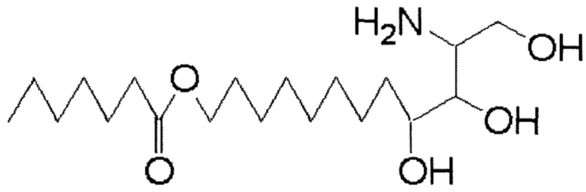

(5-4)

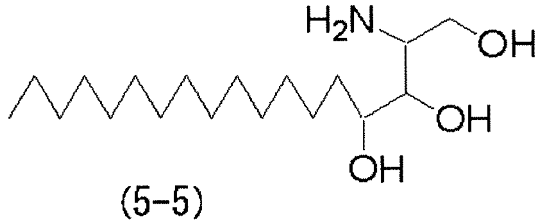

(5-5)

As phytosphingosine, either of a natural extract or a synthetic product may be used. And, phytosphingosine is available by synthesis, or as a sold product. Examples of commercially available natural sphingosines include D-Sphingosine (4-Sphingenine) (SIGMA-ALDRICH), DSphytosphingosine (DOOSAN), and phytosphingosine (Cosmofarm) and, further, the exemplified compound (5-5) is available as trade name: PHYTOSPHINGOSINE, manufactured by Evonik (formerly Deggusa).

(Acid)

When as the ceramide analogue, sphingosines are used (phytosphingosines are used according to the invention) in the topical composition for external use, it is preferable to use jointly a compound having an acidic residue capable of forming a salt with the compound. As the compound having an acidic residue, an inorganic acid, or an organic acid of a carbon number of 5 or less is preferable.

Examples of the inorganic acid include phosphoric acid, hydrochloric acid, nitric acid, sulfuric acid, perchloric acid, and carbonic acid, and phosphoric acid, and hydrochloric acid are preferable.

Examples of the organic acid include monocarboxylic acids such as formic acid, acetic acid, propionic acid, butyric acid, isobutyric acid, and valeric acid; dicarboxylic acids such as succinic acid, phthalic acid, fumaric acid, oxalic acid, malonic acid, and glutaric acid; oxycarboxylic acids such as glycolic acid, citric acid, lactic acid, pyruvic acid, malic acid, and tartaric acid; amino acids such as glutamic acid, and aspartic acid. As these compounds, phosphoric acid, hydrochloric acid, succinic acid, citric acid, lactic acid, glutamic acid, and aspartic acid are preferable, and lactic acid, glutamic acid, and aspartic acid are particularly preferable.

A jointly used acid may be used by pre-mixing with phytosphingosine, may be added at the time of formation of the ceramide analogue-containing particle, or may be used by adding as a pH adjusting agent after particle formation.

When the acid is used jointly, it is preferable that the addition amount is around 1 to 50 parts by mass relative to 100 parts by mass of phytosphingosine used.

In the topical composition for external use of the invention, only one kind ceramide analogue may be used, or two or more kinds may be used jointly, and since ceramides have generally a high melting point, and high crystallizability, it is preferable to use two or more kinds jointly from a viewpoint of emulsification stability/handling property.

In the present invention two kinds of natural ceramides are used in combination with phytosphingosine. And, it is preferable to use by combining natural ceramides, and one or more kinds selected from sugar ceramide compounds, or natural ceramides, and one or more kinds selected from phytosphingosines, from a viewpoint of finely-dividing of a particle, and improvement in dispersion stability of an emulsion.

When the latter combination is used, sphingosines are added preferably at 0.005 to 1000 parts by weight, further preferably 0.05 to 500 parts by weight, particularly preferably 0.5 to 200 parts by weight relative to 100 parts by weight of natural ceramides.

(Ceramide analogue-containing particle)

The ceramide analogue which may be used in the invention is contained in the topical composition for external use with a shape of a particle having a particle diameter of 1 nm to 50 nm, preferably 1 nm to 13 nm. Such the particle

is referred to as "ceramide analogue-containing particle" in the invention.

The ceramide analogue-containing particle may be incorporated into an external use agent after formed as a solid particle in advance, or may be formed in the system by heating the ceramide analogue into the melted state, or dissolving the ceramide analogue in an appropriate solvent to be liquid, thereafter, adding this to an aqueous phase containing a water-soluble polymer to emulsify or disperse it, thereafter, lowering a temperature to a normal temperature, or removing a solvent. For example, when other active ingredient is contained in an oily phase, since the active ingredient may be damaged under the high temperature condition, warming is performed preferably to the range of 30 to 60°C and, as described below, it is more preferable to prepare a particle by compatibilizing with other oil component, or dissolving in an organic solvent.

By adopting a particle diameter of the ceramide analogue-containing particle in the topical composition for external use of 50 nm or less, when the topical composition for external use of the invention is used in medicaments, or cosmetics, transparency of the composition is maintained, and skin absorbability becomes good.

As a method of granulating the ceramide analogue-containing particle in the minute particle diameter, the known method may be applied and, in the invention, from a viewpoint of finely-dividing, for example, it is preferable to prepare a particle using a high pressure emulsifying method of applying a high shearing force of 100 Mpa or more, or a precipitation method, or take a method using a micromixer by passing an oily phase and an aqueous phase independently through a micropath having a cross section area at a narrowest part of 1 $\mu m^2$ to 1 $mm^2$, and combining respective phases to mix them.

The particle diameter of the ceramide analogue-containing particle in the invention may be measured with a commercially available particle size distribution meter. When the topical composition for external use of the invention is, for example, an emulsion, as a method of measuring a particle size distribution of an emulsion, an optical microscope method, a cofocal laser microscope method, an electron microscope method, an atomic force microscope method, a static light scattering method, a laser diffraction method, a dynamic light scattering method, a centrifugation settling method, an electric pulse measuring method, a chromatography method, an ultrasound damping method are known, and apparatuses corresponding to each principle are commercially available.

From the viewpoint of the particle diameter range and easiness of measurement in the invention, a dynamic light scattering method is preferable in measurement of the particle diameter of the dispersed particle. Examples of a commercially available measuring apparatus using dynamic light scattering include nanotrack UPA (Nikkiso Co., Ltd.), dynamic light scattering-type particle diameter distribution measuring apparatus (trade name: LB-550, manufactured by Horiba Ltd.), and a concentrated system particle diameter analyzer (trade name: FPAR-1000, manufactured by Otsuka Electronics Co., Ltd.).

The particle diameter in the invention is a value measured using the dynamic light scattering-type particle diameter distribution measuring apparatus (trade name: LB-550, manufactured by Horiba Ltd.) and, specifically, a value measured as described below is adopted.

In the method of measuring the particle diameter, dilution is performed with pure water so that the concentration of an oil component becomes 1% by mass, and the particle diameter is measured using a quarts cell. The particle diameter may be obtained as the median diameter letting a sample refractive index to be 1.600, letting a dispersing medium refractive index to be 1.333 (pure water), and letting a viscosity of a dispersing solvent to be a viscosity of pure water.

The content of the ceramide analogue in the topical composition for external use of the invention is preferably in the range of 0.01% by mass to 5% by mass from a viewpoint of a feeling of a user, and is more preferably in the range of 0.1% by mass to 3% by mass.

(Oil component different from ceramide analogue)

The external use agent of the invention is such that the ceramide analogue-containing particle is dispersed in an aqueous phase containing the water-soluble polymer and, further, may take a form in which an oil component different from the ceramide analogue (in the present specification, arbitrarily referred to as other oil component) is contained, and the ceramide analogue is contained in the oil component.

The oil component in the invention refers to an oil component which is not separable from the ceramide analogue.

The oil component different from the ceramide analogue which is jointly used herein is not particularly limited, but may be an oil component as an active ingredient which is added depending on the use purpose of the topical composition for external use, or may be an oil component which is used for improving dispersion stability and a use feeling on a skin, or controlling physical properties of the composition.

Other oil component which may be used in the invention will be described below.

(Stenone, Sterol)

The topical composition for external use of the invention may contain at least one of stenone and sterol as other oil component. This component is useful for improving dispersion stability of the ceramide analogue-containing particle.
Examples of stenone which may be used as other oil component in the invention include the following.

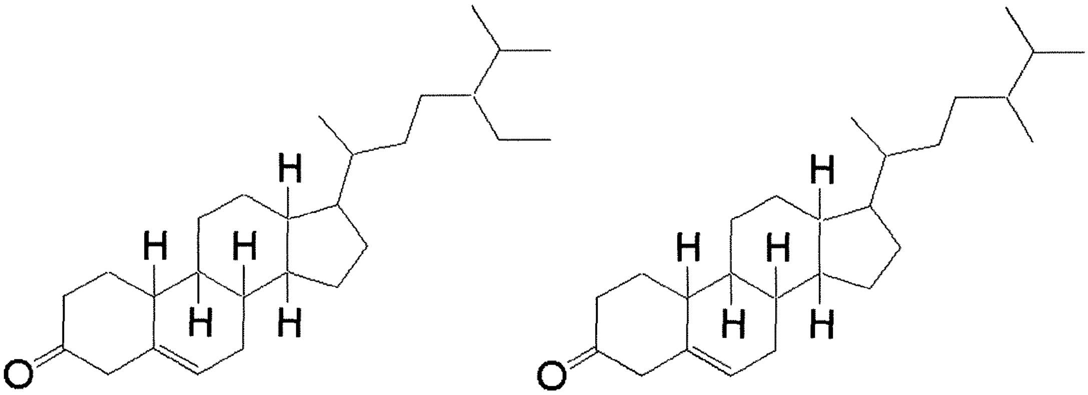

(2-1) (2-2)

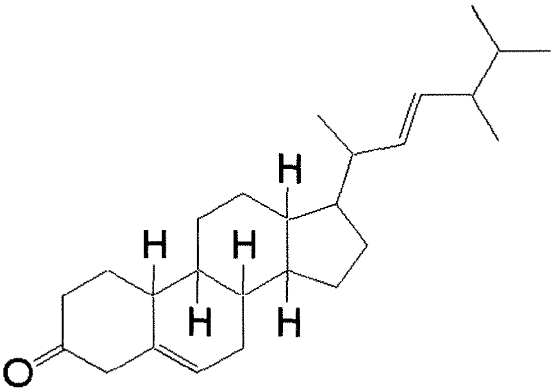

(2-3)

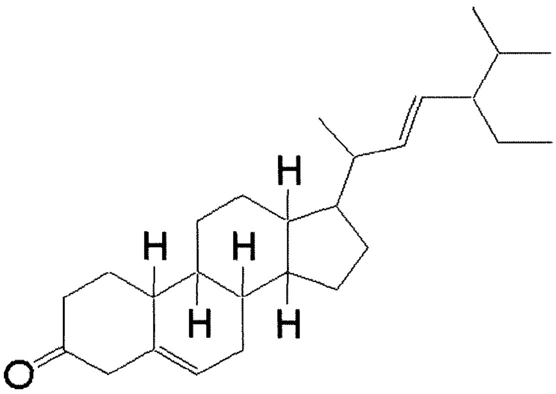

(2-4)

Examples of sterol include the following.

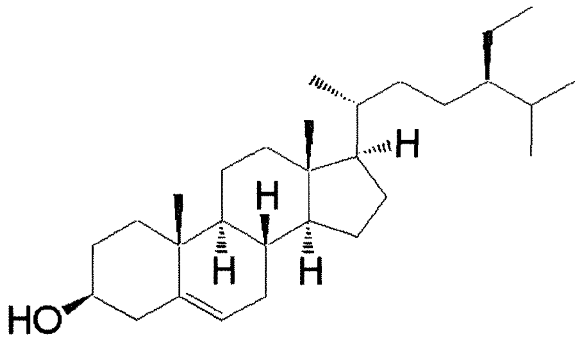

Beta-Sitosterol

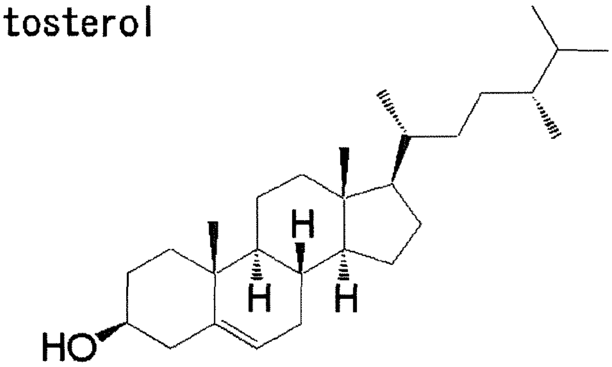

Campesterol

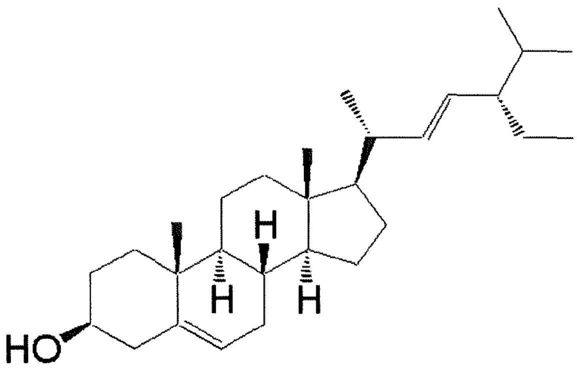

Stigmasterol

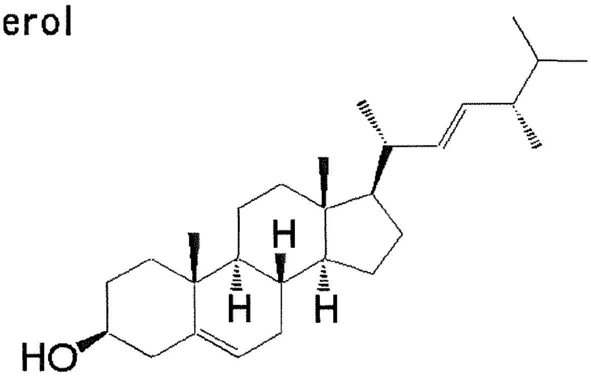

Brassicasterol

The stenone compound and the sterol compound are available by synthesis, or as a sold product.

For example, phytostenone is available as trade name: UNIFETH, manufactured by Toyohakko Co., Ltd., and

PEO-STEROL is available as trade name: NIKKOL BPS-20, manufactured by Nikko Chemicals Co., Ltd.

The stenone compound, and the sterol compound may be used alone, respectively or a plurality of kinds thereof may be used.

When the stenone compound is added as an oil component in the case where only the stenone compound is used, the content relative to the total mass of an oily phase component contained in the topical composition for external use is preferably 30% by mass to 70% by mass, more preferably 40% by mass to 60% by mass, from a viewpoint of dispersion stability of the dispersed particle.

(Oil component as active ingredient)

When the topical composition for external use of the invention is used in cosmetic utility, or medicament utility, it is preferable that a functional material for cosmetics and a functional material for medicaments which are insoluble or hardly soluble in an aqueous medium, preferably water are contained as an oil component. By inclusion of the functional oil component such as astaxanthin described later in the topical composition for external use of the invention, the excellent emollient effect, the skin aging preventing effect or the oxidation preventing effect may be imparted to the topical composition for external use of the invention.

The oil component which may be used in the invention is not particularly limited as far as it is a component which is insoluble or hardly soluble in an aqueous medium, particularly water, but a radical scavenger containing an oil-soluble vitamin such as carotenoids, and tocopherols, or fats or oils such as coconut oil are preferably used.

Insoluble in an aqueous medium refers to that solubility in 100 mL of an aqueous medium is 0.001 g or less at 25°C, and hardly soluble in an aqueous medium refers to that solubility in 100 mL of an aqueous medium is more than 0.01 g and 0.1 g or less.

(Carotenoid)

As the oil component, carotenoids including a natural colorant may be preferably used. Carotenoids which may be used in the topical composition for external use of the invention are a colorant of terpenoids from yellow to red, and include natural substances such as plants, algae, and bacteria.

In addition, carotenoids are not limited to naturally occurring carotenoids, but any carotenoids are included in carotenoid in the invention as far as they are obtained according to the conventional method. For example, many of carotenes of carotenoids described later are also produced by synthesis, and many of commercially available β-carotenes are produced by synthesis.

Examples of the carotenoids include hydrocarbons (carotenes) and oxidized alcohol derivatives thereof (xanthophylls).

Examples of carotenoids include actinioerythrol, astaxanthin, bixin, canthaxanthin, capsanthin, capsorubin, β-8'-apo-carotenal (apocarotenal), β-12'-apo-carotenal, α-carotene, β -carotene, "carotene" (mixture of α- and β- carotenes), γ-carotene, β-cryptoxanthin, lutein, lycopene, biorelithrin, and zeaxanthin, and esters of carotenoids containing hydroxyl or carboxyl among them.

Many of carotenoids are naturally present in the form of cis and trans isomers, and synthetic substances are frequently a racemic mixture.

Carotenoids may be generally extracted from plant materials. These carotenoids have various functions and, for example, lutein extracted from a petal of marigold is widely used as a raw material of a feed of poutly, and has the function of coloring a skin of poutly, and lipid, as well as an egg laid by poutly.

Carotenoids particularly preferably used in the invention is at least one of astaxanthin and a derivative such as an ester of astaxanthin which have the oxidation preventing effect, the anti-inflammatory effect, the skin aging preventing effect, and the whitening effect, and are known as a coloring material in the range of yellow to red (hereinafter, collectively referred to as "astaxanthins").

Examples of the natural material include red yeast phaffia, green alga haematococcus, marine bacteria, and britt. Other examples include an extract such as an extract from cultures thereof.

The astaxanthins may be contained in an emulsion composition of the invention, as an astaxanthin-containing oil separated/extracted (further, if necessary, arbitrarily purified) from other natural substance containing astaxanthins.

As the astaxanthins, those extracted from haematococcus alga (also referred to as haematococcus alga extract) are particularly preferable from a viewpoint of quality and productivity.

At least one of the astaxanthin and an ester thereof (astaxanthins) may be contained in the topical composition for external use of the invention, as an astaxanthin-containing oil separated/extracted from natural substances containing at least one of the astaxanthin and an ester thereof. Examples of such the astaxanthin-containing oil include extracts obtained by culturing red yeast phaffia, green alga haematococcus, or marine bacteria, and extracting from cultures thereof, and an extract from Euphausia superb.

It is known that haematococcus alga extract (haematococcus alga-derived coloring matter) is different from britt-derived from coloring matter, and synthesized astaxanthin in the kind and the content of an ester.
Astaxanthins which may be used in the invention, may be the extract, or astaxanthins obtained by arbitrarily purifying this extract if necessary, or synthetic astaxanthins.
As the astaxanthins, astaxanthins extracted from haematococcus alga (also referred to as haematococcus alga extract) are particularly preferable from a viewpoint of quality and productivity.
Examples of origins from which haematococcus alga extract which may be used in the invention is derived include Haematococcus pluvialis, Haematococcus lacustris, Haematococcus capensis, Haematococcus droebakensis, and Haematococcus zimbabwiensis.
Alternatively, in the invention, haematococcus alga extracts which are widely sold may be used, and the haematococcus alga extract may be obtained as ASTOTS-S ASTOTS-2.5 O, ASTOTS-50, and ASTOTS-100 manufactured by Takeda Shiki Co., Ltd. AstaReal Oil 50F, and AstaReal Oil 5F manufactured by Fuji Chemical Industry Co., Ltd. or BioAstin SCE7 manufactured by Toyokagaku Co., Ltd.
The content of astaxanthins in the haematococcus alga extract which may be used in the invention as a pure coloring matter is preferably 0.001 to 50% by mass, more preferably 0.01 to 25% by mass from a viewpoint of handling at production of the topical composition for external use.
The haematococcus alga extract which may be used in the invention contains astaxanthin or an ester thereof as a pure coloring matter like a coloring matter described in JP-A No.2-49091, and contains the ester generally at 50 mol % or more, preferably 75 mol % or more, more preferably 90 mol % or more.
Further detailed explanation is described in "Chemistry of Astaxanthin", 2005, Internet <URL:http://www.astaxanthin.co.jp/chemical/basic.htm>.

(Fats or oils)

Examples of fats or oils used as other oil component include fats or oils which are liquid at a normal temperature (fats or oils) and fats or oils which are solid at a normal temperature (fats).
Examples of the liquid fats or oils include an olive oil, a camellia oil, a macadamia nut oil, a castor oil, an avocado oil, an evening primrose oil, a turtle oil, a corn oil, a mink oil, a canola oil, an egg yolk oil, a sesame oil, a persic oil, a wheat germ oil, a camellia oil, a flaxseed oil, a safflower oil, a cottonseed oil, a nettle oil, a soybean oil, a peanut oil, a teaseed oil, a Japanese torreya nut oil, a rice bran oil, a paulownia furgasii oil, a paulownia tomentosa oil, a jojoba oil, germ oil, triglycerin, trioctanoic acid glycerin, triisopalmitic glycerin, a salad oil, a safflower oil, a palm oil, a coconut oil, a peanut oil, an almond oil, a hazelnut oil, a walnut oil, and a grapeseed oil.
Examples of the solid fats or oils include a beef tallow, a hardened beef tallow, a hoof oil, a beef bone oil, a mink oil, an egg yolk oil, a lard, a horse fat, a mutton suet, a hardened oil, a cacao oil, a coconut oil, a hardened coconut oil, a palm oil, a hardened palm oil, a palm oil, a Japanese wax tree oil, a wax tree nucleus oil, and a hardened castor oil.
Among them, a coconut oil which is a medium chain fatty acid triglyceride is preferably used from a viewpoint of the dispersed particle diameter and stability of the topical composition for external use.
In the invention, as the fats or oils, commercially available products may be used. And, in the invention, the fats or oils may be used alone, or may be used by mixing them.
Examples of a compound having a phenolic hydroxyl group which is other oil component which may be used in the invention include polyphenols (e.g. catechin), guaiac butter, nordihydroguaretic acid (NDGA), gallic acid esters, BHT (butylhydroxytoluene), BHA (butylhydroxyanisole), vitamin Es and bisphenols. Examples of gallic acid esters include propyl gallate, butyl gallate and octyl gallate.
Examples of the amine-based compound include phenylenediamine, diphenyl-p-phenylenediamine and 4-amino-p-diphenylamine, and diphenyl-p-phenylenediamine and 4-amino-p-diphenylamine are more preferable.
Examples of an oil-solubilized derivative of ascorbic acid and erythorbic acid include stearic acid L-ascorbyl ester, tetraisopalmitic acid L-ascorbyl ester, palmitic acid L-ascorbyl ester, palmitic acid erythorbyl ester, and tetraisopalmitic acid erythorbyl ester.
Among them, vitamin Es are particularly preferably used from a viewpoint of the excellence of stability and the function of oxidation prevention.
Vitamin Es are not particularly limited, but examples include vitamin Es selected from a compound group consisting of tocopherol and derivatives thereof, as well as a compound group consisting of tocotrienol and derivatives thereof. These may be used alone, or a plurality of them may be used together. Alternatively, vitamin E selected from a compound group consisting of tocopherol and derivatives thereof, and vitamin E selected from a compound group consisting of tocotrienol and derivatives thereof may be used by combining them.
The compound group consisting of tocopherol and derivatives thereof includes dl-$\alpha$-tocopherol, dl-$\beta$-tocopherol, dl-$\gamma$-tocopherol, dl-$\delta$-tocopherol, dl-$\alpha$-tocopherol acetate, nicotinic acid-dl-$\alpha$-tocopherol, linoleic acid-dl-$\alpha$-tocopherol, and dl-$\alpha$-tocopherol succinate. Among them, dl-$\alpha$-tocopherol, dl-$\beta$-tocopherol, dl-$\gamma$-tocopherol, dl-$\delta$-tocopherol, and a mixture

thereof (mix tocopherol) are more preferable. And, as the tocopherol derivatives, acetic acid esters of them are preferably used.

The compound group consisting of tocotrienol and derivative thereof includes α-tocotrienol, β-tocotrienol, γ-tocotrienol, and δ-tocotrienol. And, the tocotrienol derivative, acetic acid esters of them are preferably used. Tocotrienol is a compound similar to tocopherol contained in wheat varieties, rice bran, and palm oil, has three double bonds contained in a side chain part of tocopherol, and has the excellent oxidation preventing performance.

These vitamin Es are preferably contained in, particularly, an oily phase of the present topical composition for external use as an oil-soluble antioxidant because they can effectively exert the oxidation preventing function of an oil component. It is further preferable that, among the vitamin Es, at least one kind selected from the compound group consisting of tocotrienol and derivatives thereof is contained from a viewpoint of the oxidation preventing effect.

In the topical composition for external use of the invention, the content when such the other oil component is used is preferably 0.1% by mass to 50% by mass, more preferably 0.2% by mass to 25% by mass, further preferably 0.5% by mass, 10% by mass from a viewpoint of a dispersed particle diameter/emulsion stability in view of application of the topical composition for external use to medicaments and cosmetics.

When the content of the oil component is 0.1% by mass or more, since efficacy of an active ingredient may be sufficiently exerted, it becomes easy to apply the topical composition for external use to medicaments and cosmetics. On the other hand, when the content is 50% by mass or less, increase in the dispersed particle diameter and deterioration of emulsion stability are suppressed, and a stable composition is obtained.

[Surfactant]

The topical composition for external use of the invention may contain a surfactant in an oily phase.

As described above, by containing the specified stenone compound, or the specified sterol compound as other oil component in an oily phase, emulsion stability of an oily phase-dispersed particle may be improved. However, in the invention, other oily component is not necessarily required, and only the ceramide analogue-containing particle is used as a component constituting an oily phase in some cases. In such the case, use of a surfactant is useful for improving emulsion stability and dispersion stability of the dispersed particle. Even when the surfactant is used, it goes without saying that the specified stenone compound or the specified sterol compound may be used.

The surfactant in the invention is preferable in that the interface tension of oily phase/aqueous phase in the topical composition for external use may be extremely reduced and, as a result, the particle diameter may be reduced.

In the surfactant in the invention, from a viewpoint of emulsion stability, HLB of 8 or more is preferable, HLB of 10 or more is more preferable, and HLB of 12 or more is particularly preferable. An upper limit of a HLB value is not particularly limited, but is generally 20 or less, preferably 18 or less.

Herein, HLB is hydrophilicity-hydrophobicity balance which is usually used in the field of surfactants, and a calculation equation which is usually used, for example, Kawakami equation may be used. In the invention, the following Kawakami equation is adopted.

$$HLB=7+11.7 \log (M_w/M_o)$$

Wherein, $M_w$ is the molecular weight of a hydrophilic group, and $M_o$ is the molecular weight of a hydrophobic group.

Alternatively, numerical values of HLB described in catalogs may be used. And, as seen from the aforementioned equation, a surfactant of an arbitrary HLB value may be obtained by utilizing additivity of HLB.

Examples of the surfactant which may be used in the invention are not limited to, but include cationic, anionic, amphoteric, and nonionic surfactants, and nonionic surfactants are preferable. Examples of the nonionic surfactant include glycerin fatty acid ester, organic acid monoglyceride, polyglycerin fatty acid ester, propylene glycol fatty acid ester, polyglycerin condensed ricinoleic acid ester, sorbitan fatty acid ester, sucrose fatty acid ester, and polyoxyethylene sorbitan fatty acid ester. More preferable are polyglycerin fatty acid ester, sorbitan fatty acid ester, sucrose fatty acid ester, and polyoxyethylene sorbitan fatty acid ester. The surfactant is not necessarily required to be highly purified by distillation, and may be a reaction mixture.

Polyglycerin fatty acid ester used in the invention is an ester of polyglycerin of the average polymerization degree of 2 or more, preferably 6 to 15, more preferably 8 to 10, and fatty acid of a carbon number of 8 to 18 such as caprylic acid, capric acid, lauric acid, myristic acid, palmitic acid, stearic acid, oleic acid, and linoleic acid. Preferable examples of polyglycerin fatty acid ester include hexaglycerin monooleic acid ester, hexaglycerin monostearic acid ester, hexaglycerin monopalmitic acid ester, hexaglycerin monomyristic acid ester, hexaglycerin monolauric acid ester, decaglycerin monooleic acid ester, decaglycerin monostearic acid ester, decaglycerin monopalmitic acid ester, decaglycerin monomyristic acid ester, and decaglycerin monolauric acid ester.

Among them, more preferable are decaglycerin monooleic acid ester (HLB=12), decaglycerin monostearic acid ester (HLB=12), decaglycerin monopalmitic acid ester (HLB=13), decaglycerin monomyristic acid ester (HLB=14), and decaglycerin monolauric acid ester (HLB=16).

These polyglycerin fatty acid esters may be used alone, or may be used by mixing them.

Examples of the commercially available product include NIKKOL DGMS, NIKKOL DGMO-CV, NIKKOL DGMO-90V, NIKKOL DGDO, NIKKOL DGMIS, NIKKOL DGTIS, NIKKOL Tetraglyn 1-SV, NIKKOL Tetraglyn 1-O, NIKKOL Tetraglyn 3-S, NIKKOL Tetraglyn 5-S, NIKKOL Tetraglyn 5-O, NIKKOL Hexaglyn 1-L, NIKKOL Hexaglyn 1-M, NIKKOL Hexaglyn 1-SV, NIKKOL Hexaglyn 1-O, KIKKOL Hexaglyn 3-S, NIKKOL Hexaglyn 4-B, NIKKOL Hexaglyn 5-S, NIKKOL Hexaglyn 5-O, NIKKOL Hexaglyn PR-15, NIKKOL Decaglyn 1-L, NIKKOL Decaglyn 1-M, NIKKOL Decaglyn 1-SV, NIKKOL Decaglyn 1-50SV, NIKKOL Decaglyn 1-ISV, NIKKOL Decaglyn 1-O, NIKKOL Decaglyn 1-OV, NIKKOL Decaglyn 1-LN, NIKKOL Decaglyn 2-SV, NIKKOL Decaglyn 2-ISV, NIKKOL Decaglyn 3-SV, NIKKOL Decaglyn 3-OV, NIKKOL Decaglyn 5-SV, NIKKOL Decaglyn 5-HS, NIKKOL Decaglyn 5-IS, NIKKOL Decaglyn 5-OV, NIKKOL Decaglyn 5-O-R, NIKKOL Decaglyn 7-S, NIKKOL Decaglyn 7-O, NIKKOL Decaglyn 10-SV, NIKKOL Decaglyn 10-IS, NIKKOL Decaglyn 10-OV, NIKKOL Decaglyn 10-MAC, NIKKOL Decaglyn PR-20 manufactured by Nikko Chemicals Co., Ltd., Ryoto-Polyglyester L-7D, L-10D, M-10D, P-8D, SWA-10D, SWA-15D, SWA-20D, S-24D, S-28D, O-15D, O-50D, B-70D, B-100D, ER-60D, LOP-120DP, DS13W, DS3, HS11, HS9, TS4, TS2, DL15, DO13 manufactured by Mitsubishi-Kagaku Foods Corporation, Sunsoft Q-17UL, Sunsoft Q-14S, Sunsoft A-141C manufactured by Taiyo Kagaku Co., Ltd., and Poem DO-100, Poem J-0021 manufactured by Riken Vitamin.

Among them, preferable are NIKKOL Decaglyn 1-L, NIKKOL Decaglyn 1-M, NIKKOL Decaglyn 1-SV, NIKKOL Decaglyn 1-50SV, NIKKOL Decaglyn 1-ISV, NIKKOL Decaglyn 1-O, NIKKOL Decaglyn 1-OV, NIKKOL Decaglyn 1-LN, Ryoto-Polyglyester L-7D, L-10D, M-10D, P-8D, SWA-10D, SWA-15D, SWA-20D, S-24D, S-28D, O-15D, O-50D, B-70D, B-100D, ER-60D, and LOP-120DP.

Sorbitan fatty acid ester used in the invention has a carbon number of fatty acid of preferably 8 or more, more preferably 12 or more. Preferable examples of sorbitan fatty acid ester include monocaprylic acid sorbitan, monolauric acid sorbitan, monostearic acid sorbitan, sesquistearic acid sorbitan, tristearic acid sorbitan, isostearic acid sorbitan, sesquisostearic acid sorbitan, oleic acid sorbitan, sesquioleic acid sorbitan, and trioleic acid sorbitan.

These sorbitan fatty acid esters may be used alone, or may be used by mixing them.

Examples of the commercially available product include NIKKOL SL-10, SP-10V, SS-10V, SS-10MV, SS-15V, SS-30V, SI-10RV, SI-15RV, SO-10V, SO-15MV, SO-15V, SO-30V, SO-10R, SO-15R, SO-30R, and SO-15EX manufactured by Nikko Chemicals Co., Ltd., Solgen 30V, 40V, 50V, 90, and 110 manufactured by Dai-ich Kogyo Seiyaku Co., Ltd., and LeodorAS-10V, AO-10V, AO-15V, SP-L10, SP-P10, SP-S10V, SP-S30V, SP-O10V, and SP-O30V manufactured by Kao Corporation.

Sucrose fatty acid ester used in the invention has a carbon number of fatty acid of preferably 12 or more, more preferably 12 to 20.

Preferable examples of sugar fatty acid ester include sucrose dioleic acid ester, sucrose distearic acid ester, sucrose dipalmitic acid ester, sucrose dimyristic acid ester, sucrose dilauric acid ester, sucrose monooleic acid ester, sucrose monostearic acid ester, sucrose monopalmtic acid ester, sucrose monomyristic acid ester, and sucrose monolauric acid ester. Among them, sucrose monooleic acid ester, sucrose monostearic acid ester, sucrose monopalmitic acid ester, sucrose monomyristic acid ester, and sucrose monolauric acid ester are more preferable.

In the invention, these sucrose fatty acid esters may be used alone, or may be used by mixing them.

Examples of the commercially available product include Ryoto-Sugar Ester S-070, S-170, S-270, S-370, S-370F, S-570, S-770, S-970, S-1170, S-1170F, S-1570, S-1670, P-070, P-170, P-1570, P-1670, M-1695, 0-170, 0-1570, OWA-1570, L-195, L-595, L-1695, LWA-1570, B-370, B-370F, ER-190, ER-290, and POS-135 manufactured by Mitsubishi-Kagaku Foods Corporation, and DK Ester SS, F160, F140, F110, F90, F70, F50, F-A50, F-20W, F-10, F-A10E, Cosmelike B-30, S-10, S-50, S-70, S-110, S-160, S-190, SA-10, SA-50, P-10, P-160, M-160, L-10, L-50, L-160, L-150A, L-160A, R-10, R-20, O-10, and O-150 manufactured by Dai-ich Kogyo Seiyaku Co., Ltd..

Among them, preferable are Ryoto-Sugar Ester S-1170, S-1170F, S-1570, S-1670, P-1570, P-1670, M-1695, 0-1570, L-1695, DK Ester SS, F160, F140, F110, Cosmelike S-110, S-160, S-190, P-160, M-160, L-160, L-150A, L-160A, and O-150.

Polyoxyethylene sorbitan fatty acid ester used in the invention has a carbon number of fatty acid of preferably 8 or more, more preferably 12 or more. And, the length (addition mole number) of ethylene oxide of polyoxyethylene is preferably 2 to 100, more preferably 4 to 50.

Preferable examples of polyoxyethylene sorbitan fatty acid ester include polyoxyethylene monocaprylic acid sorbitan, polyoxyethylene monolauric acid sorbitan, polyoxyethylene monostearic acid sorbitan, polyoxyethylene sesquistearic acid sorbitan, polyoxyethylene trisetaric acid sorbitan, polyoxyethylene isostearic acid sorbitan, polyoxyethylene sesquiisostearic acid sorbitan, polyoxyethylene oleic acid sorbitan, polyoxyethylene sesquioleic acid sorbitan, and polyoxyethylene trioleic acid sorbitan.

These polyoxyethylene sorbitan fatty acid esters may be used alone, or may be used by mixing them.

Examples of the commercially available products include NIKKOL TL-10, NIKKOL TP-10V, NIKKOL TS-10V, NIKKOL TS-10MV, NIKKOL TS-106V, NIKKOL TS-30V, NIKKOL TI-10V, NIKKOL TO-10V, NIKKOL TO-10MV, NIKKOL TO-106V, and NIKKOL TO-30V manufactured by Nikko Chemicals Co., Ltd., Leodor TW-L106, TW-L120, TW-P120, TW-S106V, TW-S120V, TW-S320V, TW-O106V, TW-O120V, TW-O320V, TW-IS399C, Leodor Super SP-L10, and TW-L120 manufactured by Kao Corporation, and Solgen TW-20, TW-60V, and TW-80V manufactured by Dai-ich Kogyo Seiyaku Co., Ltd..

In the invention, lecithin may be used together with the water-soluble nonionic surfactant. Lecithin used in the invention has an essential constituent component of a glycerin skeleton, a fatty acid residue and a phosphoric acid residue, to which a base and a polyhydric alcohol are bound, and is also called phospholipid.

Since lecithin has a hydrophilic group and a hydrophobic group in the molecule, it has been previously used widely as an emulsifier in the fields of foods, medicaments and cosmetics.

Industrially, a substance having the lecithin purity of 60% or more is utilized as lecithin, and may be utilized in the invention and, from a viewpoint of formation of the fine oil droplet particle diameter and stability of a functional oil component, lecithin is preferably named generally as high purity lecithin, and this has the lecithin purity of 80% or more, more preferably 90% or more.

Examples of lecithin may include previously known various lecithins extracted and separated from living bodies of plants, animals and microorganisms.

Examples of such the lecithin include various lecithins derived from plants such as soybean, corn, peanut, rapeseed, wheat variety, animals such as yolk, and cow, and microorganisms such as Escherichia coli.

Examples of a chemical name of such the lecithin include glycerolecithin such as phosphatidic acid, phosphatidylglycerin, phosphatidylinositol, phosphatidylethanolamine, phosphatidylmethylethanolamine, phosphatidylcholine, phosphatidylserine, bisphosphatidic acid, and diphosphatidylglycerin (cardiolipin); sphingolecithin such as sphingomyelin.

And, in the invention, in addition to the high purity lecithin, hydrogenated lecithin, enzyme-degraded lecithin, enzyme-degraded hydrogenated lecithin, and hydroxylecithin may be used. These lecithins which may be used in the invention may be used alone, or in the form of a mixture of plural of them.

It is preferable that the amount of the surfactant in the topical composition for external use of the invention is the amount more than 0.5-fold amount based on mass relative to the total mass of oily phase components other than the surfactant (the sum of ceramide analogue-containing particle and other oil component), and more than 5-fold amount based on mass relative to the phospholipid amount. By adopting the amount of the surfactant being 0.5-fold amount or more relative to the total mass of oily phase components other than the surfactant, an emulsion of the finer particle diameter may be obtained and, by adopting the amount being 5-fold or more relative to the amount of phospholipid, it becomes difficult to deteriorate emulsion stability. Such the amount of the surfactant can make emulsion stability remarkably good, particularly when ascorbic acid, citric acid, or a salt thereof is present in the present composition.

The amount of the surfactant relative to the total mass of oily phase components other than the surfactant is preferably more than 0.5-fold amount, more preferably 2-fold amount or less, further preferably 1.5-fold amount of less, particularly preferably 1-fold amount or less, in order to obtain the fine particle diameter. By adopting the amount of the surfactant being 2-fold amount or less, this is preferable in that a problem such as more worse foaming tends to be abolished.

In addition, the amount of the surfactant relative to the amount of phospholipid is preferably more than 5-fold amount, more preferably 50-fold amount or less, further preferably 30-fold amount or less, particularly preferably 15-fold amount or less based on a mass, in order to make emulsion stability good. By adopting the amount of the surfactant being 50-fold amount or less, an amount suitable for miniaturization of a particle diameter, and emulsion stability may be obtained, and there is a tendency that occurrence of a problem such as foaming of the composition is suppressed, being preferable.

The addition amount of the surfactant is preferably 0.5 to 30% by mass, more preferably 1 to 20% by mass, further preferably 2 to 15% by mass relative to the topical composition for external use.

The addition amount of the surfactant within the above range is preferable in that the interface tension between oily phase/aqueous phase is easily decreased, stability is improved, and foaming due to addition of the surfactant may be suppressed.

[Water-soluble organic solvent]

It is preferable that the topical composition for external use of the invention contains a water-soluble organic solvent.

The water-soluble organic solvent in the invention is used as an oily phase containing a natural component for mixing with an aqueous solution described later. This aqueous organic solvent is a main component of an extracting liquid which extracts a natural component, at the same time. That is, in the invention, the natural component is used for

mixing with an aqueous solution, in the state where extracted into an extracting liquid containing the water-soluble organic solvent as a main component.

The water-soluble organic solvent used in the invention refers to an organic solvent having solubility in water at 25°C of 10% by mass or more. Solubility in water is preferably 30% by mass or more, further preferably 50% by mass or more from a viewpoint of stability of a finished emulsion or dispersion.

The water-soluble organic solvent may be used alone, or may be a mixed solvent of a plurality of water-soluble organic solvents. Alternatively, the solvent may be used as a mixture with water. When the mixture with water is used, the water-soluble organic solvent is contained preferably in an amount of at least 50% by volume or more, more preferably 70% by volume or more.

The water-soluble organic solvent is mixed with an oily phase component, is preferably used in order to prepare an oily phase, and is not included in the "oil component" in the present specification.

Examples of such the water-soluble organic solvent include methanol, ethanol, 1-propanol, 2-propanol, 2-butanol, acetone, tetrahydrofuran, acetonitrile, methyl ethyl ketone, dipropylene glycol monomethyl ether, methyl acetate, methyl acetoacetate, N-methylpyrrolidone, dimethyl sulfoxide, ethylene glycol, 1,3-butanediol, 1,4-butandiol, propylene glycol, diethylene glycol, and triethylene glycol, and a mixture thereof. Among them, when limited to utility of foods, ethanol, propylene glycol or acetone is preferable, and ethanol, or a mixed solution of ethanol and water is particularly preferable.

[Polyhydric alcohol]

It is preferable that the topical composition for external use of the invention contains a polyhydric alcohol from a viewpoint of the particle diameter, stability, and asepticus.

The polyhydric alcohol has the moisturizing function and the viscosity adjusting function. In addition, the polyhydric alcohol also has the function of reducing the interface tension between water and a fat or oil component, making an interface easily spread, and making easier to form a fine and stable particle.

From the foregoing, inclusion of the polyhydric alcohol in the topical composition for external use is preferable from a viewpoint that the dispersed particle diameter of the topical composition for external use may be finer, and the particle diameter may be stably retained for a long period of time in the state where the particle diameter is fine.

In addition, by addition of the polyhydric alcohol, the moisture activity of the topical composition for external use may be reduced, and proliferation of microorganisms may be suppressed.

The polyhydric alcohol which may be used in the invention may be used without any limitation, as far as it is a di- or more hydric alcohol.

Examples of the polyhydric alcohol include glycerin, diglycerin, triglycerin, polyglycerin, 3-methyl-1,3-butanediol, 1,3-butylene glycol, isopropylene glycol, polyethylene glycol, 1,2-pentanediol, 1,2-hexanediol, propylene glycol, dipropylene glycol, polypropylene glycol, ethylene glycol, diethylene glycol, pentaerythritol, neopentyl glycol, maltitol, reduced syrup, sucrose, lactitol, paratinit, erythritol, sorbitol, mannitol, xylitol, xylose, glucose, lactose, mannose, maltose, galactose, fructose, inositol, pentaerythritol, maltotriose, sorbitan, trehalose, starch-degraded sugar, and starch-degraded sugar reducing alcohol, and these may be used alone, or in the form of a mixture of plural kinds.

It is preferable to use a polyhydric alcohol having the number of hydroxyl groups in one molecule of 3 or more. Thereby, the interface tension between an aqueous solvent and a fat or oil component may be more effectively reduced, and a finer, and stable particle may be formed. As a result, in the case of food utility, intestinal tract absorbability may be enhanced and, in the case of transdermal medicament utility and cosmetic utility, skin absorbability may be enhanced.

Among polyhydric alcohols satisfying the aforementioned conditions, particularly when glycerin is used, the oil droplet particle diameter of the topical composition for external use becomes more smaller, and the particle is stably retained for a long period of time while the particle diameter is small, being preferable.

The content of the polyhydric alcohol is preferably 10 to 60% by mass, more preferably 20 to 55% by mass, further preferably 30 to 50% by mass relative to the topical composition for external use, from a viewpoint of the viscosity of the topical composition for external use in addition to the particle diameter, the stability and the acepticus.

When the content of the polyhydric alcohol is 10% by mass or more, this is preferable in that sufficient storage stability is easily obtained depending on the kind and the content of the fat or oil component. On the other hand, when the content of the polyhydric alcohol is 60% by mass or less, this is preferable in that the maximum effect is obtained, and increase in the viscosity of the topical composition for external use is easily suppressed.

In the topical composition for external use of the invention, if necessary, other additives which are usually used in the topical composition for external use, such as various drug efficacy ingredients, antiseptic, and coloring agent may be used together in such the range that the effect of the invention is not deteriorated.

[Other component]

In addition to the aforementioned components, if necessary, components used in the topical composition for external use such as a skin external use agent may be arbitrarily used depending on the purpose.
Examples of such the compound include a moisturizing agent such as glycinebetaine, xylitol, trehalose, urea, neutral amino acid, and basic amino acid, a drug efficacy agent such as allantoin, and tocopheryl acetate, an organic powder such as cellulose powder, nylon powder, crosslinked silicone powder, crosslinked methylpolysiloxane, porous cellulose powder, and porous nylon powder, an inorganic powder such as anhydrous silica, zinc oxide, and titanium oxide, a refreshing agent such as menthol and camphor, a plant extract, a pH buffer solution, an antioxidant, a ultraviolet absorbing agent, an antiseptic, a perfume, a fungicide, and a coloring matter.
In the topical composition for external use, when the ceramide analogue-containing particle is used together with other oil component in the oily phase, a finely-divided oily phase particle (containing ceramide analogue-containing particle) having the particle diameter of an oily phase of objective 0.2 μm or less depending on the stirring condition (shearing force, temperature, pressure), the condition of using a micromixer, and a ratio of an oily phase and an aqueous phase in a process for producing a topical composition for external use described later, in addition to factors due to the aforementioned components of the topical composition for external use, may be obtained.

<Process for producing topical composition for external use>

The topical composition for external use of the invention comprises a ceramide analogue-containing particle and a water-soluble polymer and, generally, takes the form in which the ceramide analogue-containing particle, or an oily phase formed with other oil component and the ceramide analogue is dispersed in an aqueous phase containing the water-soluble polymer.
A process for producing such the dispersion comprises preparing an oily phase containing the ceramide analogue, or an oily phase containing the ceramide analogue and other oil component, and an aqueous phase containing the water-soluble polymer separately, and emulsifying prepared oily phase and aqueous phase to form the ceramide analogue-containing particle in an aqueous phase containing the water-soluble polymer.
Thereupon, it is preferable that the viscosity of an aqueous phase containing the water-soluble polymer is 30 mPa·s or less, from a viewpoint of finely-dividing of the ceramide analogue-containing particle.
In the step of preparing the ceramide analogue-containing particle by this emulsification, it is preferable that the separately prepared oily phase and aqueous phase are each independently passed through a micropath having the cross-sectional area of a narrowest part of 1 $\mu m^2$ to 1 $mm^2$ and, thereafter, respective phases are combined and mixed, from a viewpoint of fine dividing. The preparation temperature may be changed depending on a boiling point of a solvent used and, usually, preparation is implemented at preferably 20°C to 80°C, more preferably 20°C to 60°C. In addition, a method of mixing separately prepared oily phase and aqueous phase as described above, and applying a high pressure emulsifying method of adding a high shearing force such as 100 MPa or more is preferably exemplified.
In such a way, a topical composition for external use in which ceramide analogue-containing particles having the volume average particle diameter of 1 nm to 50 nm are dispersed may be obtained.
Examples of the process for producing the topical composition for external use of the invention include a) preparing an aqueous phase containing a water-soluble polymer using an aqueous medium (water etc.), b) mixing the ceramide analogue in at least 1% by mass relative to the total mass of an oily phase and, optionally, a water-soluble organic solvent, the specified stenone compound and other oil component (carotenoid etc.) to prepare an oily phase, and c) mixing the oily phase and the aqueous phase using a micromixer by a method detailed later to perform emulsification and dispersing, to obtain a topical composition for external use (emulsion) containing a dispersed particle having the volume average particle diameter of 1 nm to 50 nm.
When one wants to obtain the topical composition for external use of the invention in the powder state, the topical composition for external use in the powder state may be obtained by adding a step of drying a ceramide dispersion in the emulsion state obtained above by spray drying, and the like.
Components contained in the oily phase and the aqueous phase in the aforementioned production process are the same as constituent components of the topical composition for external use of the invention as described above, a preferable example and a preferable amount are also the same, and a preferable combination is more preferable.
The ratio (mass) of the oily phase and the aqueous phase in the emulsification and dispersing is not particularly limited, but is preferably 0.1/99.9 to 50/50, more preferably 0.5/99.5 to 30/70, further preferably 1/99 to 20/80 expressed as oily phase/aqueous phase ratio (mass %).
By adopting the oily phase/aqueous phase ratio in the above range, an active ingredient is sufficiently contained, and practically sufficient emulsion stability is obtained, being preferable.

[Micromixer]

In the process for producing the topical composition for external use of the invention, it is preferable to take a process of passing the oily phase and the aqueous phase each independently through a mircopath having the cross-sectional area of a narrowest part of 1 $\mu m^2$ to 1 $mm^2$, and combining and mixing respective phases for the purpose of stably containing the ceramide analogue-containing particle of 0.2 $\mu$m or less as defined in the invention.

The mixing of the oily phase and the aqueous phase is preferably mixing by countercurrent collision from a viewpoint of obtaining the finer dispersed particle.

The most suitable device for mixing by countercurrent collision is a countercurrent collision-type micromixer. The micromixer mixes mainly two different liquids in a fine space, one of liquids is an organic solvent phase containing a functional oil component, and the other is an aqueous phase which is an aqueous solution.

When the micromixer is applied to preparation of an emulsion having the small particle diameter which is one of microchemistry processes, a good emulsion or dispersion having relatively low energy and small heat production, having the more uniform particle diameter as compared with a normal stirring emulsification dispersing system or high pressure homogenizer emulsification dispersing, and also having the excellent storage stability is easily obtained. This is an optimal method for emulsifying a natural component which is easily thermally degraded.

A summary of a method of emulsification or dispersing using the micromixer include dividing the aqueous phase and the oily phase into fine spaces, respectively, and contacting or colliding respective fine spaces. This method is clearly different from a membrane emulsification method or a microchannel emulsification method which is a method in which only one is divided into a fine space, and the other is a bulk and, even when only one is actually divided into a fine space, the effect as in the invention is not obtained. As the known micromixer, there are a variety of structures. When attention is paid to flow and mixing in a micropath, there are two kinds of a method of mixing while a laminar flow is maintained, and a method of mixing while disturbed, that is, in a disturbed flow. In the method of mixing while a laminar flow is maintained, mixing is effectively performed by making a size of a path depth greater than a path width, thereby, increasing the area of an interface between two liquids as much as possible, and making thicknesses of both layers smaller. Alternatively, a method of adopting a multilayer flow by dividing an entrance for two liquids into many potions, and flowing two liquids alternately has been also devised.

On the other hand, in a method of mixing with the disturbed flow, a method of flowing respective flows at a relatively high speed by dividing them into narrow paths is general. A method of ejecting one of fluids into the other liquid introduced into a fine space using an arrayed micronozzle has been also proposed. Alternatively, a method of forcibly contacting liquids flowing at a high speed using various means is good, particularly in the mixing effect. In the former method using a laminar flow, generally, a produced particle is large, and distribution is relatively uniform, on the other hand in the latter method using a disturbed flow, there is a possibility that a very fine emulsion is obtained. In respect of stability and transparency, the method using a disturbed flow is preferable in many cases. As the method using a disturbed flow, a comb tooth type and a collision type are representative. The comb tooth type micromixer has a structure in which two comb tooth-like paths are faced, and arranged so that one path enters between two the other paths, alternately a representative of which is a mixer manufactured by IMM.

In the invention, the micromixer shown in JP-A No.2004-33901 may be also preferably used.

Fig. 2 is a schematic cross-sectional view of T-type microreactor, showing one example of a mixing mechanism with a T-type microreactor. Fig. 3 is a conceptional view of a T-type microreactor, showing one example of a mixing mechanism with a T-type microreactor.

In Fig. 2, a cross-section of a T-type path 200 of a T-type microreactor is shown. In the T-type path 200, a fluid which has been flown therein in a direction of an arrow D through an inlet 202a, and a fluid which has been flown therein in a direction of an arrow E through an inlet 202b are collided at a central part in a path of the T-type path 200, and mixed to become a fine fluid particle. The fine fluid particle is flown out in a direction of an arrow F through an outlet 204. This T-type microreactor is useful for mixing when the volume of a path is small.

In Fig. 3, a fluid mixing mechanism (concept) 300 of other T-type microreactor is shown. In the fluid mixing mechanism shown in Fig. 3, fluids which have been flown therein through two paths 302a and 302b are mutually collided and mixed to become a fine fluid particle. That is, the fluid, on one hand, is flown in a path 302a in a direction of an arrow G, and is flown out in a direction of an arrow H. On the other hand, the fluid is flown in a path 302b in a direction of an arrow I, and is flown out in a direction of an arrow J. Fluids which have been flown out through paths 302a and 302b, respectively, are collided, are mixed, and are flied approximately orthogonal with a direction of an arrow G to J. The fluid mixing mechanism described in the path figure, Fig. 3, collides and mixes fluids diffused by a procedure of misting. By this collision and mixing, the fluid becomes finer, and a great contact surface may be obtained.

The collision-type micromixer, a representative which is the KM mixer, has a structure in which forcible contact is tried utilizing the kinetic energy. Specifically, there is a central collision-type micromixer disclosed by Nagasawa et al. ("H.Nagasawa et al., Chem.Eng.Technol.,28,No.3,324-330 (2005)", JP-A No.2005-288254). In the method of countercurrently colliding an aqueous phase and an organic solvent phase, since a mixing time is extremely short, and an oily

phase droplet is instantly formed, an extremely fine emulsion or dispersion is easily formed.

In the invention, when emulsification is performed by micro-mixing with the collision-type micromixer, a temperature at emulsification (emulsification temperature) is such that micro-mixing is performed at a temperature of the aforementioned separate fine space of the micromixer (temperature at micro-mixing part of micromixer) at preferably 80°C or lower, more preferably 0°C to 80°C, particularly preferably 5°C to 75°C, from a viewpoint of particle diameter uniformity of the resulting emulsion. By adopting the emulsification temperature of 0°C or higher, since a main component of a dispersing medium is water, the emulsification temperature may be managed, being preferable. A retained temperature of the fine space of the micromixer is preferably 100°C or lower. By adopting the retained temperature of 100°C or lower, management of the retained temperature may be easily controlled, and the micro-bumping phenomenon which adversely influences on emulsification performance may be excluded. It is further preferable that the retained temperature is controlled at a temperature of 80°C or lower.

Retained temperatures of the oily phase and the aqueous phase divided into the fine space of the micromixer, and of the fine space of the micromixer are different depending on components contained in the aqueous phase and the oily phase, and are each independently preferably 0°C to 50°C, particularly preferably 5°C to 25°C. The retained temperature of the fine space of the micromixer, the retained temperatures of the oily phase and aqueous phase divided into the fine space of the micromixer, and the retained temperatures of the oily phase and the aqueous phase before division into the fine space of the micromixer (i.e. retained temperatures of oily phase and aqueous phase supplying tanks) may be different, respectively, but the same temperature is preferable in stability of mixing.

In the invention, it is particularly preferable that retained temperatures of the aqueous phase and the oily phase before and after division into the fine space of the micromixer, and of the fine space of the micromixer and the separate fine space are higher than room temperature and, after micro-mixing and emulsification, an oil-in-water emulsion obtained with the micromixer is cooled to a normal temperature after collection.

The cross-sectional area of a narrowest part of the fine space (path) of the micromixer in the invention is 1 $\mu m^2$ to 1 $mm^2$ and, from a viewpoint of miniaturization of the emulsion particle diameter and sharpness of the particle diameter distribution, preferably 500 $\mu m^2$ to 50,000 $\mu m^2$.

The cross-sectional area of a narrowest part of the fine space (path) of the micromixer used in the aqueous phase in the invention is particularly preferably 1,000 $\mu m^2$ to 50,000 $\mu m^2$ from a viewpoint of mixing stability.

The cross-sectional area of a narrowest part of the fine space (path) of the micromixer used in the oily phase is particularly preferably 500 $\mu m^2$ to 20,000 $\mu m^2$ from a viewpoint of miniaturization of the emulsion particle diameter and sharpness of the particle diameter distribution.

When emulsification and dispersing are performed with the micromixer, the flow rate of the oily phase and the aqueous phase at emulsification and dispersing are different depending on the micromixer used and, from a viewpoint of miniaturization of the emulsion particle diameter and sharpness of the particle diameter distribution, the flow rate of the aqueous phase is preferably 10 ml/min to 500 ml/min, more preferably 20 ml/min to 350 ml/min, particularly preferably 50 ml/min to 200 ml/min.

The flow rate of the oily phase, from a viewpoint of miniaturization of the emulsion particle diameter and sharpness of the particle diameter distribution, is preferably 1 ml/min to 100 ml/min, more preferably 3 ml/min to 50 ml/min, particularly preferably 5 ml/min to 50 ml/min.

The value obtained by dividing flow rates of both phases by the cross-sectional area of a microchannel, that is, the flow speed ratio (Vo/Vw) of both phases is preferably in the range of 0.05 or more and 5 or less from a viewpoint of miniaturization of a particle and design of the micromixer, wherein Vo is the flow speed of an organic solvent phase containing a water-insoluble natural component, and Vw is the flow speed of an aqueous phase. And, the flow speed ratio (Vo/Vw) of 0.1 or more and 3 or less is the most preferable range from a viewpoint of further miniaturization of a particle.

In addition, solution sending pressures of the aqueous phase and the oily phase are preferably 0.030 MPa to 5 MPa and 0.010 MPa to 1 MPa, more preferably 0.1 MPa to 2 MPa and 0.02 MPa to 0.5 MPa, particularly preferably 0.2 MPa to 1 MPa and 0.04 MPa to 0.2 MPa, respectively. By adopting the solution sending pressure of the aqueous phase of 0.030 MPa to 5 MPa, the stable solution sending flow rate tends to be maintained and, by adopting the solution sending pressure of the oily phase of 0.010 MPa to 1 MPa, uniform mixing property tends to be obtained, being preferable.

In the invention, the flow rate, the solution sending pressure and the retained temperature are more preferably a combination of respective preferably examples.

Then, a route from introduction of the aqueous phase and the oily phase into the mixromixer to discharge as a oil-in-water droplet emulsion will be explained using an example of a microdevice (Fig. 1) as one example of the mixromixer in the invention.

As shown in Fig. 1, a microdevice 100 is constructed of a supply element 102, a confluence element 104 and a discharge element 106, each in a cylindrical form.

On a surface opposite to the confluence element 104 of the supply element 102, a cross-section as a path for the oily phase or the aqueous phase in the invention is such that rectangular annular channels 108 and 110 are concentrically formed. In the supply element 102, bores 112 and 114 leading to each annular channel are formed, penetrating in a direction of its thickness (or height) direction.

In the confluence element 104, a bore 116 penetrating in its thickness direction is formed. In this bore 116, when an element is secured thereto in order to construct the microdevice 100, an end 120 of the bore 116 situated on a surface of the confluence element 104 opposite to the supply element 102 is opened in the annular channel 108. In an embodiment shown, four bores 116 are formed, and they are arranged at an equal interval in a circumferential direction of the annular channel 108.

In the confluence element 104, a bore 118 is formed, penetrating therethrough, like the bore 116. The bore 118 is formed so as to be opened in the annular channel 110, like the bore 116. Bores 118 are arranged at an equal interval in a circumferential direction of the annular channel 110, and the bore 116 and the bore 118 are arranged so as to be positioned alternately.

On a surface 122 opposite to the discharge element 106 of the confluent element 104, the microchannels 124 and 126 are formed. One end of this microchannel 124 or 126 is an opening part of the bore 116 or 118, the other end is a center 128 of the surface 122, and all microchannels extend from bores towards this center 128, and are converged at a center. A cross-section of the microchannel may be, for example, rectangular.

In the discharge element 106, a bore 130 passing a center thereof and penetrating in a thickness direction is formed. Therefore, this bore is opened in the center 128 of the confluence element 104 at one end, and is opened in the outside of the microdevice at the other end.

In the present microdevice 100, fluids A and B supplied from the outside of the microdevice 100 at ends of bores 112 and 114 are flown into annular channels 108 and 110 via bores 112 and 114, respectively.

The annular channel 108 and the bore 116 are communicated, and the fluid A which has flown into the annular channel 108 enters a microchannel 124 via the bore 116. In addition, the annular channel 110 and the bore 118 are communicated, and the fluid B which has flown into the annular channel 110 enters a microchannel 126 via the bore 118. Fluids A and B are flown into microchannels 124 and 126, respectively, and are flown towards a center 128, and are converged.

The converged fluids are discharged as a stream C to the outside of the microdevice via the bore 130.

Such the microdevice 100 may have the following spec.

Cross-sectional shape of annular channel 108, width/depth/diameter: rectangular, 1.5/1.5/25 mm
Crosse-sectional shape of annular channel 110, width/depth/diameter: rectangular, 1.5/1.5/20 mm
Diameter and length of bore 112: 1.5/10 mm (circular cross-section)
Diameter and length of bore 114: 1.5/10 mm (circular cross-section)
Diameter and length of bore 116: 0.5/4 mm (circular cross-section)
Diameter and length of bore 118: 0.5/4 mm (circular cross-section)
Cross-sectional shape of microchannel 124, width, depth, length: rectangular, cross-sectional area, 350 $\mu$m/100 $\mu$m/12.5 mm/35000 $\mu m^2$
Cross-sectional shape of microchannel 126, width, depth, length: rectangular, cross-sectional area, 50 $\mu$m/100 $\mu$m/10 mm/5000 $\mu m^2$
Diameter and length of bore 130: 500 $\mu$m, 10 mm (circular cross-section)

The size of the microchannel (in Fig. 1, 124 and 126) in which the aqueous phase and the oily phase are collided is defined in the preferable range in context with flow rates of the aqueous phase and the oily phase.

In the production process of the invention, it is preferable that a water-soluble organic solvent which has been used is removed after emulsification and dispersing through the micropath. As a method of removing a solvent, an evaporation method using a rotary evaporator, a flash evaporator, or an ultrasound atomizer, and a membrane separating method such as an ultrafiltration membrane and a reverse osmosis membrane are known, and an ultrafiltration membrane method is particularly preferable.

An ultra filter (abbreviated as UF) is an apparatus by which a stock solution (water, mixed aqueous solution of high-molecular substance, low-molecular substance, and colloidal substance) is pressurized, and water is poured into a UF apparatus, thereby, the stock solution may be separated into two-system solutions of a permeated solution (low-molecular substance) and a concentrated solution (high-molecular substance, colloidal substance), and taken out.

The ultrafiltration membrane is a typical asymmetric membrane made by the Leob-Sourirajan method. A polymer material used includes polyacrylonitrile, polyvinyl chloride-polyacrylonitrile copolymer, polysulfone, polyether sulfone, vinylidene fluoride, aromatic polyamide, and cellulose acetate. Recently, a ceramic membrane has become to be used. Unlike a reverse osmosis method, in an ultrafiltration method, since pre-treatment is not performed, fouling occurs, in which a polymer is deposited on a membrane surface. For this reason, it is normal to wash the membrane with a chemical or warm water periodically. For this reason, a membrane material is required to have resistance to a chemical and heat resistance. As a membrane module of an ultrafiltration membrane, there are various kinds such as flat membrane type, tubular type, hollow thread type, and spiral type. An index for performance of an ultrafiltration membrane is a fractionation molecular weight, and various membranes having a fractionation molecular weight of 1,000 to 300,000 are commercially

available. As the commercially available membrane module, there are Microsa UF (Asahi Kasei Chemicals Corporation), and capillary-type element (trade name: NTU-3306, manufactured by Nitto Denko Corporation), being not limiting.

For removing a solvent from the emulsion related to the invention, a material of a membrane is particularly preferably polysulfone, polyether sulfone, and aromatic polyamide are particularly preferable from a viewpoint of solvent resistance. As the form of a membrane module, a flat membrane is mainly used at a laboratory scale, and a hollow shred type and spiral type are industrially used, and a hollow shred type is particularly preferable. In addition, a fraction molecular weight is different depending on a kind of an active ingredient and, usually, the range of 5,000 to 100,000 is used.

An operation temperature may be 0°C to 80°C and, in view of degradation of an active ingredient, the range of 10°C to 40°C is particularly preferable.

As an ultrafiltration device at a laboratory scale, there are ADVANTEC-UHP (ADVANTEC), Flow Type Labotest Unit RUM-2 (Nitto Denko Corporation) using and a flat membrane-typed module. Industrially, respective membrane modules at the size and the number depending on the necessary potency may be arbitrarily combined to construct a plant. As a bench scale unit, RUW-5A (Nitto Denko Corporation) is commercially available.

In the production process of the invention, a step of concentrating the resulting emulsion subsequent to solvent removal may be added. As the concentrating method, the same method and the device as those of solvent removal such as an evaporation method and a filtration membrane method may be used. Also in the case of concentration, an ultrafiltration membrane method is a preferable method. When the same membrane as that of solvent removal may be used, this is preferable and, if necessary, ultrafiltration membranes having different fractionation molecular weights may be also used. Alternatively, a concentration efficacy may be enhanced by operating at a temperature different from that of solvent removal.

The topical composition for external use (emulsion) obtained by mixing with the micromixer is a oil-in-water droplet emulsion. In the process for producing the topical composition for external use of the invention, the volume average particle diameter (median diameter) of the dispersed particle of the emulsion is 1 nm to 50 nm from a viewpoint of transparency of the resulting emulsion.

The particle diameter of the dispersed particle obtained by the process for producing the topical composition for external use of the invention may be measured with a commercially available particle size distribution meter, and details thereof are as described above.

<Utility>

The topical composition for external use of the invention is a fine emulsion composition excellent in the emollient effect due to the ceramide compound. For this reason, the composition is preferably used in a variety of utilities depending on the function of the ceramide compound.

As such the utility, the composition may be widely used, for example, in medicaments (external use preparations, skin preparations), or cosmetics. Examples of the medicament include parenteral preparations such as suppositories, and coating preparations (skin external use preparations), and examples of the cosmetic include skin care cosmetics (lotions, beauty essences, emulsions, creams, etc.), sun-screen cosmetics, and makeup cosmetics such as lip rouges and foundations, being not limiting.

When the cosmetic for external use of the invention is used in skin external use preparations, and cosmetics, if necessary, components which may be added to medicaments and cosmetics may be arbitrarily added.

When the topical composition for external use of the invention is used in beauty washes, beauty essences, emulsions, cream packs/masks, packs, hair washing cosmetics, fragrance cosmetics, liquid body cleansing preparations, UV care cosmetics, deodorant cosmetics, oral care cosmetics, analgesics or antiphlogstic-containing gels, and aqueous products such as drug efficacy ingredient-containing layer of antiphlogistic-containing patch, products having a transparency feeling are obtained, and occurrence of inconvenient phenomenon such as settlement, precipitation or neckling of insolubles under the severe conditions such as long term storage and sterilization treatment may be suppressed.

## EXAMPLES

The present invention will be further specifically explained below by way of Examples, but the invention is not limited to the following Examples as far as it is not departed from the gist thereof. Unless otherwise is indicated, "part" is on a mass basis.

(Examples 1-A to 1-F, Comparative Example 1-G)

Respective components described in the following oily phase liquid 1 composition were stirred at room temperature for 1 hour to prepare an oily phase liquid 1.

<Oily phase liquid 1 composition>

| | |
|---|---|
| Ceramide 3 [Ceramide compound, embodiment 1-5] | 0.1 part |
| Ceramide 6 [Ceramide compound, embodiment 1-7] | 0.1 part |
| Phytosphingosine | 0.07 part |
| Ethanol [Water-soluble organic solvent] | 150 parts |

IN hydrochloric acid (adjusted so that a pH immediately after dispersing became 7 or lower)

The resulting oily phase liquid 1 (oily phase) and water (aqueous phase) were micro-mixed at the ratio (mass ratio) of 1:7 using a KM-type micromixer 100/100 which is an collision type, to obtain an emulsion (ceramide-dispersed composition) 1.

The condition for using the micromixer are as follows.

- Microchannel-

Oily phase side microchannel

Cross-sectional phase/width/depth/length=rectangular/70 μm/100 μm/10 mm

Aqueous phase side microchannel

Cross-sectional phase/width/depth/length=rectangular/490 μm/100 μm/10 mm

-Flow rate-

An aqueous phase was introduced into an external annulus at the flow rate of 21.0 ml/min, an oil phase was introduced into an internal annulus at the flow rate of 3.0 ml/min, and these were micro-mixed.

The resulting emulsion (ceramide-dispersed composition) was desolvated to the ethanol concentration of 0.1% or less using Evapor (CEP-lab) manufactured by Ogawara Corporation, and this was concentrated and adjusted to the emulsion concentration of 2.0% to obtain an emulsion A. The emulsion concentration referred herein is the concentration based on the sum of a solid matter added to an oily phase.

Then, respective components described in the following Table 1 were stirred at room temperature for 5 hours to prepare addition liquids 1-A to 1-F.

Each of the resulting addition liquids 1-A to 1-F at 50 parts was taken, and added to 50 parts of the emulsion (ceramide-dispersed composition) 1 obtained from the oily phase liquid 1, and the mixture was stirred with a magnetic stirrer at 300 rpm for 30 minutes to obtain topical compositions for external use 1-A to 1-F having the final emulsion concentration of 1.0%.

Separately, 50 g of water were added to 50 g of the emulsion (ceramide-dispersed composition) 1 to prepare a comparative external use preparation 1-G according to the same method.

[Table 1]

| Water-soluble polymer (Average molecular weight) | Addition liquid 1-A | Addition liquid 1-B | Addition liquid 1-C | Addition liquid 1-D | Addition liquid 1-E | Addition liquid 1-F |
|---|---|---|---|---|---|---|
| PEG3000 (3,000) | 0.5 | - | - | - | - | - |
| Hydrolyzed collagen (3,000) | - | 0.23 | - | - | - | - |
| Hyaluronic acid (one million) | - | - | 0.6 | - | - | - |

(continued)

| Water-soluble polymer (Average molecular weight) | Addition liquid 1-A | Addition liquid 1-B | Addition liquid 1-C | Addition liquid 1-D | Addition liquid 1-E | Addition liquid 1-F |
|---|---|---|---|---|---|---|
| Hyaloronic acid (60,000) | - | - | - | 0.6 | - | - |
| Xanthan gum (two million) | - | - | - | - | 0.8 | - |
| Dextran (70,000) | - | - | - | - | - | 0.8 |
| Total amount (adjusted with water) | 1000 | 1000 | 1000 | 1000 | 1000 | 1000 |
| Viscosity (mPa.s)(25°C) | 1 | 3 | 60 | 5 | 20 | 6 |

(Examples 2-A to 2-F, Comparative Example 2-G)

The following components were stirred at room temperature for 1 hour to prepare an oily phase liquid 2.

| | |
|---|---|
| Ceramide 3 [Ceramide compound, embodiment 1-5] | 0.1 part |
| Ceramide 6 [Ceramide compound, embodiment 1-7] | 0.1 part |
| Phytosphingosine | 0.03 part |
| Ethanol [water-soluble organic solvent] | 150 parts |

IN hydrochloric acid (adjusted so that a pH immediately after dispersing became 7 or lower)

Then, respective components described in the following Table 2 were stirred at room temperature for 5 hours to prepare addition liquids 2-A to 2-F (unit: part).

The resulting oily phase liquid 2 (oily phase) and each of addition liquids 2-A to 2-F as an aqueous phase were micro-mixed at the ratio (mass ratio) of 1:7 using a KM-type micromixer 100/100 which is a collision type, to obtain compositions for external use 2-A to 2-F.

Separately, according to the same manner except that 50 parts of water was added to the oily phase liquid 2 in place of each of the respective addition liquids, a comparative external use preparation 2-G was prepared.

The conditions for using the micromixer are as follows.

-Microchannel-

Oily phase side microchannel

$$\text{Cross-sectional shape/width/depth/length=rectangular/70 }\mu\text{m/100 }\mu\text{m/10 mm}$$

Aqueous phase side microchannel

$$\text{Cross-sectional shape/width/depth/length=rectangular/490 }\mu\text{m/100 }\mu\text{m/10 mm}$$

-Flow rate-

The aqueous phase was introduced into an external annulus at the flow rate of 21.0 ml/min, the oily phase was introduced into an internal annulus at the flow rate of 3.0 ml/min, and this was micro-mixed.

The resulting emulsion (ceramide-dispersed composition) was desolvated to the ethanol concentration of 0.1% or less using Evapor (CEP-lab) manufactured by Okawara Corporation, and this was concentrated and adjusted to the

emulsion concentration of 1.0% to obtain Example compositions for external use 2-A to 2-F and a comparative topical composition for external use 2-G.

The emulsion concentration referred herein is the concentration based on the sum of a solid matter added to the oily phase.

[Table 2]

| Water-soluble polymer (Average molecular weight) | Addition liquid 2-A | Addition liquid 2-B | Addition liquid 2-C | Addition liquid 2-D | Addition liquid 2-E | Addition liquid 2-F |
|---|---|---|---|---|---|---|
| PEG3000 (3,000) | 0.5 | - | - | - | - | - |
| Hydrolyzed collagen (3,000) | - | 0.23 | - | - | - | - |
| Hyaluronic acid (one million) | - | - | 0.23 | - | - | - |
| Hyaluronic acid (60,000) | - | - | - | 0.23 | - | - |
| Xanthan gum (two million) | - | - | - | - | 0.23 | - |
| Dextran (70,000) | - | - | - | - | - | 0.23 |
| Total amount (adjusted with water) | 1000 | 1000 | 1000 | 1000 | 1000 | 1000 |
| Viscosity (mPa·s) (25°C) | 1 | 3 | 20 | 5 | 15 | 6 |

<Evaluation>

1. Particle diameter of ceramide analogus-containing particle

The particle diameter of the ceramide analogus-containing particle in the crude topical composition for external use immediately after preparation, or an oily phase particle containing the ceramide analogus-containing particle was measured using a dynamic light scattering-type particle diameter distribution measuring apparatus (trade name: LB-550, manufactured by Horiba Ltd.).

Measurement of the particle diameter was performed using a quartz cell by performing diluting the composition for the external use with pure water so that the concentration of the ceramide analogue-containing particle became 1 % by mass. The particle diameter may be obtained as the median diameter when a sample refractive index was set at 1.600, a dispersing medium refractive index was set at 1.333 (pure water), and the viscosity of a dispersing medium was set at the viscosity of pure water.

2. Evaluation of stability with time of sample

Stability with time was evaluated by the following method using the turbidity. The turbidity immediately after preparation of each emulsified composition of compositions for external use 1-A to 1-F, and 2-A to 2-F of Examples, and the comparative compositions for external use 1-G, and 2-G was measured as an absorbance at 600 nm with a 10 mm cell using UV-VIBLE spectrophotometer (trade name: UV-2550, manufactured by Shimadzu Corporation) (temperature 25°C).

Further, 7 cycles (2 weeks) of repetition of storing each sample in a constant temperature chamber at 60°C for 24 hours and, then, storing in a refrigerator at 4°C for 24 hours were performed. After that, a temperature was returned to 25°C, the turbidity was measured again, and a difference between the turbidity immediately after preparation was compared, and evaluation was performed according to the following criteria. Results are shown in the following Table 3.

D: Change in turbidity 0.1 or more (disapproved from viewpoint of merchandize value)
C: Change in turbidity 0.05 to less than 0.1 (barely acceptable from viewpoint of merchandize value)
B: Change in turbidity 0.01 to less than 0.05 (change is seen, but no problem from viewpoint of merchandize value)
A: Change in turbidity less than 0.01 (it is difficult to percept change visually)

[Table 3]

| | Ceramide analogue-containing particle (μm) | External preparation dispersing stability | |
|---|---|---|---|
| | | Δ Turbidity | Evaluation |
| Example 1-A | 0.023 | 0.094 | C |
| Example 1-B | 0.023 | 0.063 | C |
| Example 1-C | 0.023 | 0.022 | B |
| Example 1-D | 0.023 | 0.012 | B |
| Example 1-E | 0.023 | 0.034 | B |
| Example 1-F | 0.023 | 0.028 | B |
| Comparative Example 1-G | 0.023 | 0.15 | D |
| Example 2-A | 0.025 | 0.085 | B |
| Example 2-B | 0.023 | 0.048 | B |
| Example 2-C | 0.020 | 0.015 | B |
| Example 2-D | 0.025 | 0.008 | A |
| Example 2-E | 0.022 | 0.024 | B |
| Example 2-F | 0.026 | 0.009 | A |
| Comparative Example 2-G | 0.023 | 0.15 | D |

As apparent from the Table 3, it is seen that the topical composition for external use of the invention has the small particle diameter of the ceramide analogue-containing particle, and is excellent in dispersibility and dispersing stability of such the particle.

In comparison between Examples 1-A to 1-F and Examples 2-A to 2-F, it is seen that the effect of the invention is more improved in the topical composition for external use obtained by a production process of separately preparing the oily phase and the aqueous phase and, thereafter, mixing and emulsifying them.

Further, topical compositions for external use 1-A' to 1-F' and a comparative topical composition for external use 1-G' were prepared in the same manner as Examples 1-A to 1-F and Comparative Example 1-G except that ceramide 3 was changed to ceramide 3B. In addition, topical compositions for external use 2-A' to 2-F' and a comparative topical composition for external use 2-G' were prepared in the same manner as Examples 2-A to 2-F and Comparative Example 2-G except that ceramide 3 was changed to ceramide 3B.

The particle diameter of the ceramide analogus-containing particle in the topical compositions for external use 1-A' to 1-F', 2-A' to 2-F', and comparative topical compositions for external use 1-G' and 2-G' were measured in the same manner as of Examples 1-A to 1-F, 2-A to 2-F and Comparative Examples 1-G and 2-G. Further, stability with time was evaluated in the same manner according to the same criteria as of Examples 1-A to 1-F, 2-A to 2-F and Comparative Examples 1-G and 2-G.

Table of the results is not shown, however, the results were similar to those of Examples 1-A to 1-F, 2-A to 2-F and Comparative Examples 1-G and 2-G. It is also seen that the topical composition for external use of the invention, that is, each of the topical compositions for external use 1-A' to 1-F' and 2-A' to 2-F' has the small particle diameter of the ceramide analogue-containing particle, and is excellent in dispersibility and dispersing stability of such the particle.

## Claims

1. A topical composition for external use comprising: a ceramide analogue-containing particle having a particle diameter of 1 nm to 50 nm, and a water-soluble polymer, wherein
   the ceramide analogue is a combination of phytosphingosine and two kinds of natural ceramides

2. The topical composition for external use according to claim 1, further comprising an oil component different from the ceramide analogue in an amount of 20 parts by mass or less relative to 1 part by mass of the ceramide analogue-containing particle.

3. The topical composition for external use according to claim 2, wherein the content of the oil component different from the ceramide analogue-containing particle is 10 parts by mass or less relative to 1 part by mass of the ceramide analogue-containing particle.

4. The topical composition for external use according to claim 1, wherein the water-soluble polymer is a natural polymer.

5. The topical composition for external use according to claim 1, wherein the water-soluble polymer is a collagen derivative, wherein the collagen derivative is at least one selected from a hydrolyzed collagen or a water-soluble collagen.

6. The topical composition for external use according to claim 5, wherein the weight-average molecular weight of the collagen derivative is 5,000 or less.

7. The topical composition for external use according to claim 1, wherein the water-soluble polymer is a polysaccharide.

8. The topical composition for external use according to claim 7, wherein the weight average molecular weight of the polysaccharide is 100,000 or less.

9. The topical composition for external use according to claim 1, wherein the water-soluble polymer is hyaluronic acid.

10. The topical composition for external use according to claim 9, wherein the weight average molecular weight of the hyaluronic acid is 300,000 or less.

11. The topical composition for external use according to claim 1, wherein the ceramide analogue-containing particle is formed in the presence of the water-soluble polymer.

12. A process for producing a topical composition for external use containing a ceramide analogue-containing particle having a particle diameter of 1 nm to 50 nm; and a water-soluble polymer, the process comprising forming a ceramide analogue-containing particle in an aqueous phase containing a water-soluble polymer, wherein the ceramide analogue isa combination of phytosphingosine and two kinds of natural ceramides.

13. The process for producing a topical composition for external use according to claim 12, wherein the viscosity of the aqueous phase is 30 mPa·s or less.

**Patentansprüche**

1. Topische Zusammensetzung zur äußeren Anwendung umfassend: einen Ceramidanalogon-enthaltenden Partikel mit einem Partikeldurchmesser von 1 nm bis 50 nm und ein wasserlösliches Polymer, wobei das Ceramidanalogon eine Kombination aus Phytosphingosin und zwei natürlichen Ceramiden ist.

2. Topische Zusammensetzung zur äußeren Anwendung nach Anspruch 1, ferner umfassend eine von dem Ceramidanalogon unterschiedliche Ölkomponente in einer Menge von 20 Masseanteilen oder weniger bezogen auf 1 Masseanteil des Ceramidanalogon-enthaltenden Partikels.

3. Topische Zusammensetzung zur äußeren Anwendung nach Anspruch 2, wobei der Gehalt an der von dem Ceramidanalogon-enthaltenden Partikel unterschiedlichen Ölkomponente 10 Masseanteile oder weniger bezogen auf 1 Masseanteil des Ceramidanalogon-enthaltenden Partikels ist.

4. Topische Zusammensetzung zur äußeren Anwendung nach Anspruch 1, wobei das wasserlösliche Polymer ein natürliches Polymer ist.

5. Topische Zusammensetzung zur äußeren Anwendung nach Anspruch 1, wobei das wasserlösliche Polymer ein Kollagenderivat ist, wobei das Kollagenderivat mindestens eines, ausgewählt aus einem hydrolysierten Kollagen oder einem wasserlöslichen Kollagen ist.

6. Topische Zusammensetzung zur äußeren Anwendung nach Anspruch 5, wobei das gewichtsgemittelte Molekulargewicht des Kollagenderivats 5000 oder weniger beträgt.

7. Topische Zusammensetzung zur äußeren Anwendung nach Anspruch 1, wobei das wasserlösliche Polymer ein Polysaccharid ist.

8. Topische Zusammensetzung zur äußeren Anwendung nach Anspruch 7, wobei das gewichtsgemittelte Molekulargewicht des Polysaccharids 100000 oder weniger beträgt.

9. Topische Zusammensetzung zur äußeren Anwendung nach Anspruch 1, wobei das wasserlösliche Polymer Hyaluronsäure ist.

10. Topische Zusammensetzung zur äußeren Anwendung nach Anspruch 9, wobei das gewichtsgemittelte Molekulargewicht der Hyaluronsäure 300000 oder weniger beträgt.

11. Topische Zusammensetzung zur äußeren Anwendung nach Anspruch 1, wobei der Ceramidanalogon-enthaltende Partikel in der Anwesenheit des wasserlöslichen Polymers gebildet wird.

12. Verfahren zur Herstellung einer topischen Zusammensetzung zur äußeren Anwendung enthaltend einen Ceramidanalogon-enthaltenden Partikel mit einem Partikeldurchmesser von 1 nm bis 50 nm; und ein wasserlösliches Polymer, wobei das Verfahren ein Formen eines Ceramidanalogon-enthaltenden Partikels in einer ein wasserlösliches Polymer enthaltenden wässrigen Phase umfasst, wobei das Ceramidanalogon eine Kombination aus Phytosphingosin und zwei natürlichen Ceramiden ist.

13. Verfahren zur Herstellung einer topischen Zusammensetzung zur äußeren Anwendung nach Anspruch 12, wobei die Viskosität der wässrigen Phase 30 mPa·s oder weniger beträgt.

**Revendications**

1. Composition topique pour une utilisation externe, comprenant : une particule contenant un analogue de céramide présentant un diamètre de particules allant de 1 nm à 50 nm, et un polymère hydrosoluble, dans laquelle l'analogue de céramide est une combinaison de phytosphingosine et de deux types de céramides naturels.

2. Composition topique pour une utilisation externe selon la revendication 1, comprenant en outre un composant huileux différent de l'analogue de céramide en une quantité allant de 20 parties en masse ou moins par rapport à 1 partie en masse de la particule contenant un analogue de céramide.

3. Composition topique pour une utilisation externe selon la revendication 2, dans laquelle la teneur en composant huileux différent de la particule contenant un analogue de céramide est inférieure ou égale à 10 parties en masse par rapport à 1 partie en masse de la particule contenant un analogue de céramide.

4. Composition topique pour une utilisation externe selon la revendication 1, dans laquelle le polymère hydrosoluble est un polymère naturel.

5. Composition topique pour une utilisation externe selon la revendication 1, dans laquelle le polymère hydrosoluble est dérivé de collagène, dans laquelle le dérivé de collagène est au moins un élément sélectionné parmi un collagène hydrolysé ou un collagène hydrosoluble.

6. Composition topique pour une utilisation externe selon la revendication 5, dans laquelle le poids moléculaire moyen en poids du dérivé de collagène est inférieur ou égal à 5000.

7. Composition topique pour une utilisation externe selon la revendication 1, dans laquelle le polymère hydrosoluble est un polysaccharide.

8. Composition topique pour une utilisation externe selon la revendication 7, dans laquelle le poids moléculaire moyen en poids du polysaccharide est inférieur ou égal à 100 000.

**9.** Composition topique pour une utilisation externe selon la revendication 1, dans laquelle le polymère hydrosoluble est de l'acide hyaluronique.

**10.** Composition topique pour une utilisation externe selon la revendication 9, dans laquelle le poids moléculaire moyen en poids de l'acide hyaluronique est inférieur ou égal à 300 000.

**11.** Composition topique pour une utilisation externe selon la revendication 1, dans laquelle la particule contenant un analogue de céramide est formée en présence du polymère hydrosoluble

**12.** Procédé destiné à produire une composition topique pour une utilisation externe contenant une particule contenant un analogue de céramide présentant un diamètre de particules allant de 1 nm à 50 nm, et un polymère hydrosoluble, le procédé comprenant la formation d'une particule contenant un analogue de céramide dans une phase aqueuse contenant un polymère hydrosoluble, dans lequel l'analogue de céramide est une combinaison de phytosphingosine et de deux types de céramides naturels.

**13.** Procédé destiné à produire une composition topique pour une utilisation externe selon la revendication 12, dans lequel la viscosité de la phase aqueuse est inférieure ou égale à 30 mPa·s.

FIG.1

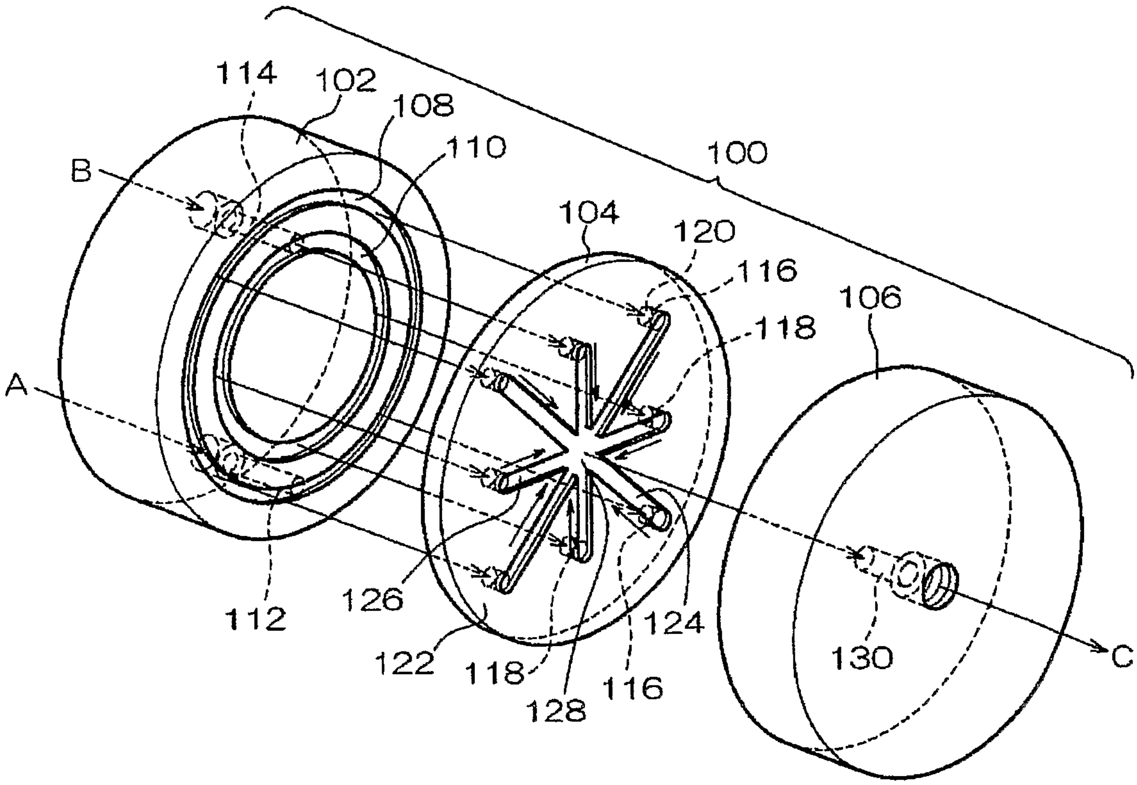
114
102
108
110
100
B
104
120
116
118
106
A
112
126
124
122
118
128
116
130
C

FIG.2

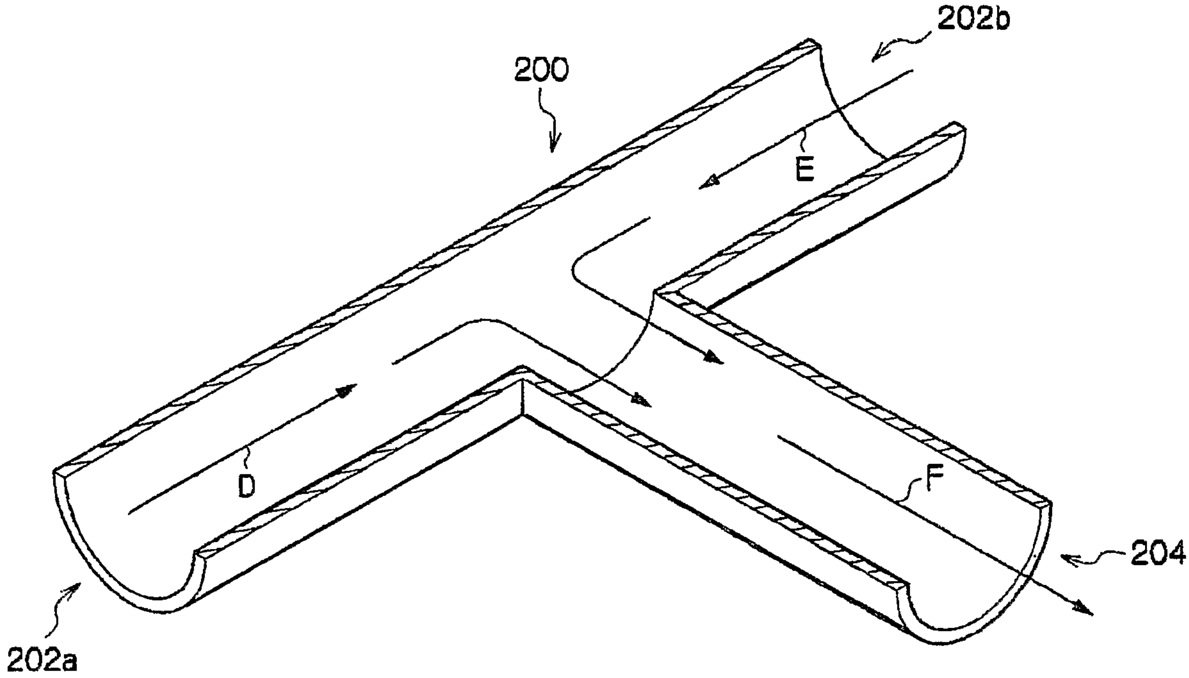

FIG.3

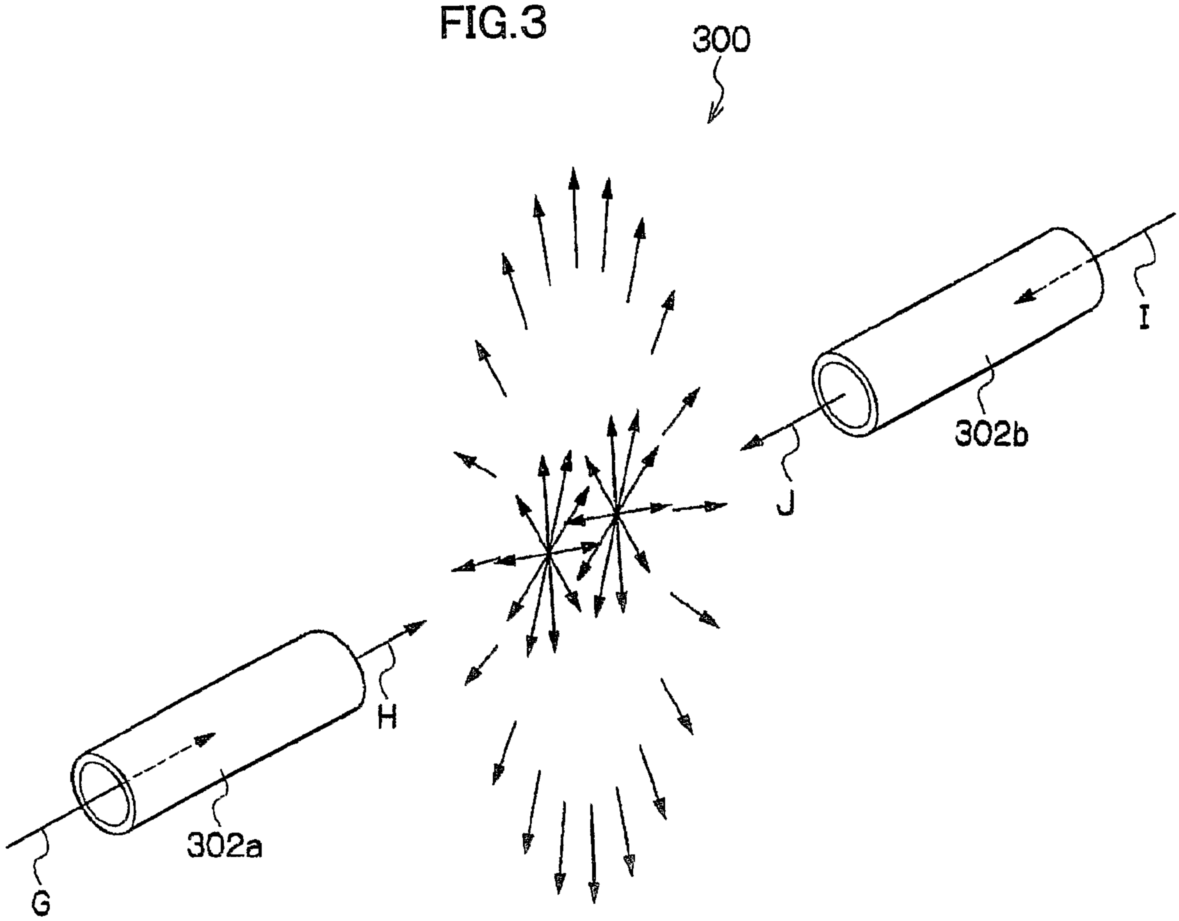

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2000051676 A
- JP 7187987 A
- JP 2006335692 A
- JP 11310512 A
- JP 2001139796 A
- JP 2001316217 A
- JP 2049091 A
- JP 2004033901 A
- JP 2005288254 A


### Non-patent literature cited in the description


- PHYTOSPHINGOSINE (INCI name
- *Chemistry of Astaxanthin,* 2005, http://www.astaxanthin.co.jp/chemical/basic.htm
- **H.NAGASAWA et al.** *Chem.Eng.Technol.,* 2005, vol. 28 (3), 324-330